(19) Europäisches Patentamt
European Patent Office
Office européen des brevets

(11) EP 1 193 260 B1

(12) # EUROPEAN PATENT SPECIFICATION

(45) Date of publication and mention of the grant of the patent:
**07.12.2005 Bulletin 2005/49**

(51) Int Cl.7: **C07D 265/38**, C07D 265/34, A61K 31/538, A61P 25/28

(21) Application number: **01122733.7**

(22) Date of filing: **21.09.2001**

(54) **Phenoxazine analogs for the treatment of amyloidosis**

Phenoxazinanaloge zur Behandlung von Amyloidose

Analogues de phenoxazine pour le traitement d'amyloidose

(84) Designated Contracting States:
**AT BE CH CY DE DK ES FI FR GB GR IE IT LI LU MC NL PT SE TR**

(30) Priority: **29.09.2000 US 236966 P**

(43) Date of publication of application:
**03.04.2002 Bulletin 2002/14**

(73) Proprietor: **Warner-Lambert Company LLC**
**Morris Plains, NJ 07950 (US)**

(72) Inventors:
- **Augelli-Szafran, Corinne Elizabeth, Pfizer Global Ann Arbor, Michigan 48105 (US)**
- **Lai, Yingije Edison, New Jersey 08817 (US)**
- **Yasunaga, Tomoyuki, c/oYamanouchi Pharm. Co. Ltd. Tokyo 103-8411 (JP)**

(74) Representative: **Tesch, Rudolf et al**
**Warner-Lambert Company,**
**Legal Division,**
**Patent Department,**
**Mooswaldallee 1**
**79090 Freiburg (DE)**

(56) References cited:
**WO-A-00/76489** **WO-A-00/76969**
**WO-A-96/30766**

- **C. M. WISCHIK ET AL.: "Selective inhibition of Alzheimer disease-like tau aggregation by phenothiazines" PROC. NATL. ACAD. SCI., vol. 93, 1996, pages 11213-11218, XP002067057 USA**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 193 260 B1

## Description

FIELD OF THE INVENTION

This invention relates to compounds useful for inhibiting amyloid protein aggregation and imaging amyloid deposits. In addition, this invention relates to a method of treating Alzheimer's disease and disorders related to amyloidosis.

SUMMARY OF THE RELATED ART

Amyloidosis is a condition characterized by the accumulation of various insoluble, fibrillar proteins in the tissues of a patient. The fibrillar proteins that comprise the accumulations or deposits are called amyloid proteins. While the particular proteins or peptides found in the deposits vary, the presence of fibrillar morphology and a large amount of β-sheet secondary structure is common to many types of amyloids. An amyloid deposit is formed by the aggregation of amyloid proteins, followed by the further combination of aggregates and/or amyloid proteins.

The presence of amyloid deposits has been shown in various diseases, each with its particular associated protein, such as Mediterranean fever, Muckle-Wells syndrome, idiopathetic myeloma, amyloid polyneuropathy, amyloid cardiomyopathy, systemic senile amyloidosis, hereditary cerebral hemorrhage with amyloidosis, Alzheimer's disease, Down syndrome, scrapie, Creutzfeldt-Jakob disease, kuru, Gerstmann-Sträussler-Scheinker syndrome, medullary carcinoma of the thyroid, isolated atrial amyloid, $\beta_2$-microglobulin amyloid in dialysis patients, inclusion body myositis, $\beta_2$-amyloid deposits in muscle wasting disease, sickle cell anemia, Parkinson's discase, and Islets of Langerhans diabetes type 2 insulinoma.

Alzheimer's disease is a degenerative brain disorder characterized clinically by progressive loss of memory, cognition, reasoning, judgement, and emotional stability that gradually leads to mental deterioration and ultimately death. Because Alzheimer's disease and related degenerative brain disorders are a major medical issue for an increasingly aging population, the need for new treatments and methods for diagnosing the disorders are needed.

A simple, noninvasive method for detecting and quantitating amyloid deposits in a patient has been eagerly sought. Presently, detection of amyloid deposits involves histological analysis of biopsy or autopsy materials. Both methods have major drawbacks. For example, an autopsy can only be used for a postmortem diagnosis.

The direct imaging of amyloid deposits in vivo is difficult, as the deposits have many of the same physical properties (i.e., density and water content) as normal tissues. Attempts to image amyloid deposits directly using magnetic resonance imaging (MRI) and computer-assisted tomography (CAT) have been disappointing and have detected amyloid deposits only under certain favorable conditions. In addition, efforts to label amyloid deposits with antibodies, serum amyloid P protein, or other probe molecules has provided some selectivity on the pcriphery of tissues, but has provided for poor imaging of tissue interiors.

Thus, it would be useful to have a noninvasive technique for imaging and quantitating amyloid deposits in a patient. In addition, it would be useful to have compounds that inhibit the aggregation of amyloid proteins to form amyloid deposits. An object of this invention is to provide new compounds that are useful to diagnose and treat diseases associated with amyloidosis.

SUMMARY OF THE INVENTION

The present invention provides compounds that are useful in a method of inhibiting amyloid protein aggregation, the method comprising the administration of an effective amount of the compound to a subject, preferably mammalian, in need thereof.

The present invention is directed to phenoxazine derivatives and their use as inhibitors of amyloid protein aggregation. The compounds of the invention are those having the structure of Formula I

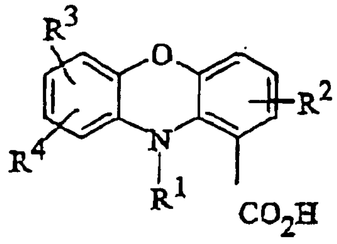

I

and pharmaceutically acceptable salts, esters, wherein the esters are selected from $C_1$-$C_6$ alkyl esters, $C_5$-$C_7$ cycloalkyl esters or arylalkyl esters wherein arylalkyl is defined as below and amides wherein the amides are selected from amides derived from ammonia, primary $C_1$-$C_6$ alkyl amines and secondary $C_1$-$C_6$ dialkyl amines wherein the alkyl groups are straight or branched chain, in the case of secondary amines, the amine may also be in the form of a 5- or 6-membered heterocycle containing one nitrogen atom,
thereof,
wherein:

$R^1$ is
hydrogen,
$C_1$-$C_6$ alkyl which can be substituted with one or more of the substituents listed below for aryl, or
cycloalkyl, wherein cycloalkyl means a carbocyclic ring having from 3 to 7 carbon atoms which may be substituted with one or more of the substituents listed below for aryl.
$R^2$ is
hydrogen;
$C_1$-$C_6$ alkyl,
$C_1$-$C_6$ alkoxy,
halogen,
hydroxy
aryl, wherein aryl means phenol, biphenyl, 1,2,3,4-tetrahydronaphthyl, naphthyl, anthryl, or phenanthryl, unsubstituted or substituted by 1 to 3 substituents selected from alkyl, O-alkyl and S-alkyl, OH, SH, -CN, halogen, 1,3-dioxolanyl, $CF_3$, $NO_2$, $NH_2$, $NHCH_3$, $N(CH_3)_2$, NHCO-alkyl, $-(CH_2)_mCO_2H$, $-(CH_2)_mCO_2$-alkyl, $-(CH_2)_mSO_3H$, -NH alkyl-N(alkyl)$_2$, $-(CH_2)_mPO_3H_2$, $-(CH_2)_mPO_3(alkyl)_2$, $-(CH_2)_mSO_2NH_2$, and-$(CH_2)_mSO_2$NH-alkyl wherein alkyl is defined as above and m is 0, 1, 2, or 3,
heteroaryl, wherein heteroaryl means thienyl, furanyl, thiazolyl, imidazolyl, (is)oxazolyl, pyridyl, pyrimidinyl, (iso)quinolinyl, naphthyridinyl, benzimidazolyl, benzoxazolyl, and heterocycle is unsubstituted or substituted by 1 to 3 substituents selected from alkyl, O-alkyl and S-alkyl, OH, SH, -CN, halogen, 1,3-dioxolanyl, $CF_3$, $NO_2$, $NH_2$, $NHCH_3$, $N(CH_3)_2$, NHCO-alkyl, $-(CH_2)_mCO_2H$, $-(CH_2)_mCO_2$-alkyl,$-(CH_2)_mSO_3H$, -NH alkyl, -N(alkyl)$_2$, $-(CH_2)_mPO_3H_2$,$-(CH_2)_mPO_3(alkyl)_2$, $-(CH_2)_mSO_2NH_2$, and $-(CH_2)_mSO_2$NH-alkyl wherein alkyl is defined as above and m is 0, 1, 2, or 3, arylalkyl, wherein arylalkyl means an alkyl moiety defined as above substituted with an aryl moiety also defined as above,
hateroarylalkyl, wherein heteroaryl and alkyl are defined as above, arylalkoxy, wherein aryl and alkoxy are defined as above, heteroarylalkoxy, wherein heteroaryl and alkoxy are defined as above, cyano,
carboxy,
alkoxycarbonyl, wherein alkoxy is defined as above,
carbamoyl,
sulfamoyl,
nitro,
trifluoromethyl,
amino, or mono- or dialkylamino wherein alkyl is defined as above;
$R^3$ and $R^4$ independently are
hydrogen,
$C_1$-$C_6$ alkoxy,
aryl defined as above,
heteroaryl defined as above,
halogen,
hydroxy,
cyano,
carboxy,
alkoxycarbonyl defined as above,
carbamoyl,
sulfamoyl,
nitro,
trifluoromethyl,
amino, mono- or dialkylamino defined as above, or
$C_1$-$C_6$ alkyl or alkenyl having from 1 to 6 carbon atoms unsubstituted or substituted with one, two or three groups independently selected from oxo, halogen, hydroxy, carboxy, carbamoyl, amino, mono- or dialkylamino defined as above, or

aryl defined as above or heteroaryl defined as above optionally substituted independently with up to three groups selected from halogen, $C_1$-$C_6$ alkyl, $C_1$-$C_6$ alkoxy, hydroxy, carboxy, alkoxycarbonyl defined as above, cyano, nitro, trifluoromethyl, amino, mono- or dialkylamino defined as above, carbamoyl, carboxyalkyl defined as above, alkoxycarbonylalkyl defined as above, sulfamoyl, or carbonylamino, or

$R^3$ and $R^4$together form a carbocyclic group containing from five to seven members, up to two of which members are optionally heteroatoms selected from oxygen and nitrogen, where the carbocyclic group is optionally substituted with one or two groups selected from halogen, $C_1$-$C_6$ alkyl,

$C_1$-$C_6$ alkoxy, mono- or dialkylamino defined as above, aryl defined as above, arylalkyl defined as above, or a heterocyclic group defined as above.

The instant invention includes pharmaceutical compositions of compounds of Formula I and a method of treating Alzheimer's disease, the method comprising administering to a patient having Alzheimer's disease a therapeutically effective amount of a compound of Formula I. Also provided is a method for treating disorders related to amyloidosis, the method comprising administering to a patient having disorders related to amyloidosis a therapeutically effective amount of a compound of Formula I.

In a further embodiment, radiolabeled compounds of Formula I are provided, as well as a method for detecting and quantitating amyloid deposits by administering such radiolabeled compound to an animal and measuring the localization thereof in tissues.

Also provided is a method of inhibiting the aggregation of amyloid proteins to form amyloid deposits, the method comprising administering to a patient in need of inhibition of the aggregation of amyloid protein an amyloid protein aggregation inhibiting amount of a compound of Formula I.

In a further embodiment, radiolabeled compounds of Formula I are provided, as well as a method for detecting and quantitating amyloid deposits by administering such radiolabeled compound to an animal and measuring the localization there of in tissues.

DETAILED DESCRIPTION OF THE INVENTION

The novel compounds encompassed by the instant invention are those described by the general Formula I set forth above, and the pharmaceutically acceptable salts, esters, and amides thereof.

Preferred compounds of Formula I are those in which $R^1$ is hydrogen; $R^2$ is hydrogen, nitro, or amino; $R^3$ is hydrogen, hydroxy, trifluoromethyl, halogen, or nitro; and $R^4$ is halogen, aryl, or arylalkyl.

In addition to the compounds of Formula I, the invention encompasses compounds of Formula II

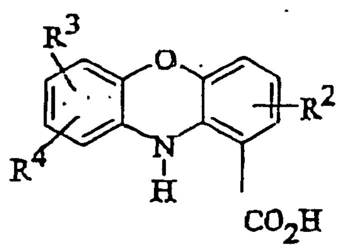

II

wherein $R^2$, $R^3$, and $R^4$ are as defined above for Formula I.

Preferred compounds of Formula II are those in which $R^2$ is hydrogen, nitro, or amino; $R^3$ is hydrogen, hydroxy, trifluoromethyl, halogen, or nitro; and $R^4$ is halogen, aryl, or arylalkyl.

In addition, the invention encompasses compounds of Formula III

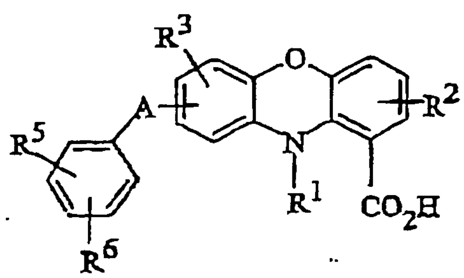

III

wherein:

$R^1$, $R^2$, and $R^3$ are as defined above for Formula I;
$R^5$ and $R^6$ are as defined above for $R^2$ in Formula II; and
A is absent, or is
lower alkyl or lower alkenyl unsubstituted or substituted with one or two groups independently selected from oxo, halogen, hydroxy, carboxy, carbamoyl, amino, mono- or dialkylamino.

Preferred compounds of Formula III are those in which $R^1$ is hydrogen; $R^2$ is hydrogen, nitro or, amino; $R^3$ is hydrogen, hydroxy, trifluoromethyl, halogen, or nitro; $R^5$ is hydrogen or halogen; and $R^6$ is hydrogen or halogen.
In addition, the invention encompasses compounds of Formula IV:

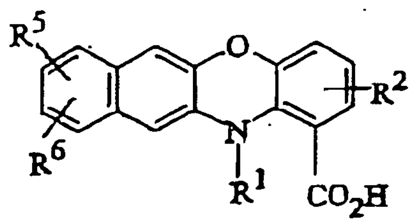

IV

wherein $R^1$, $R^2$, $R^5$, and $R^6$ are as defined above for Formula I.
Preferred compounds of Formula IV are those in which $R^1$ is hydrogen; $R^2$ is hydrogen, nitro, or amino; $R^5$ is hydrogen, lower alkyl, hydroxy, or halogen; and $R^6$ is hydrogen, lower alkyl, hydroxy, or halogen.
Another preferred group of compounds are those of Formula I wherein $R^1$ is alkyl.
Except as expressly defined otherwise, the following definition of terms is employed throughout this specification.
The terms "alkyl", "lower alkyl", or "(C1-C6)-alkyl" mean a straight or branched hydrocarbon having from 1 to 6 carbon atoms and includes, for example, methyl, ethyl, n-propyl, isopropyl, n-butyl, sec-butyl, isobutyl, tert-butyl, n-pentyl, n-hexyl, and the like. The alkyl group can also be substituted with one or more of the substituents listed below for aryl.
By "alkoxy," "lower alkoxy," or "(C1-C6)-alkoxy" in the present invention is meant straight or branched chain alkoxy groups having 1 to 6 carbon atoms, such as, for example, methoxy, ethoxy, propoxy, isopropoxy, n-butoxy, sec-butoxy, tert-butoxy, pentoxy, 2-pentyl, isopentoxy, neopentoxy, hexoxy, 2-hexoxy, 3-hexoxy, and 3-methylpentoxy.
The term "cycloalkyl" means a carbocyclic ring having from 3 to 7 carbon atoms. Examples include cyclopropyl, cyclopentyl, and cycloheptyl. The rings may be substituted with one or more of the substituents listed below for aryl. Examples include 2-aminocyclobutyl.
The term "halogen" includes chlorine, fluorine, bromine, and iodine, and their monovalent radicals.
The term "aryl" means an aromatic carbocyclic group having a single ring that is phenyl, multiple rings that is biphenyl or multiple condensed rings in which at least one is aromatic that are 1,2,3,4-tetrahydronaphthyl, naphthyl, anthryl, or phenanthryl, unsubstituted or substituted by 1 to 3 substituents selected from alkyl, O-alkyl and S-alkyl, OH, SH, -CN, halogen, 1,3-dioxolanyl, $CF_3$, $NO_2$, $NH_2$, $NHCH_3$, $N(CH_3)_2$, NHCO-alkyl, $-(CH_2)_mCO_2H$, $-(CH_2)_mCO_2$-alkyl, $-(CH_2)_mSO_3H$, -NH alkyl, $-N(alkyl)_2$, $-CH_2)_mPO_3H_2$, $-(CH_2)_mPO_3(alkyl)_2$, $-(CH_2)_mSO_2NH_2$, and $-(CH_2)_mSO_2NH$-alkyl wherein alkyl is defined as above and m is 0, 1, 2, or 3. A preferable aryl group of the present invention is phenyl. Typical substituted phenyl groups include 2-chlorophenyl, 3-methoxyphenyl, 4-aminophenyl, 3,5-dinitrophenyl, 2,6-dibromo-4-ethoxyphenyl, and 2-hydroxy-3-cyano-5-trifluoromethylphenyl.
The term "aralkyl" or "arylalkyl" means an alkyl moiety (as defined above) substituted with an aryl moiety (also as defined above).
By heteroaryl (aromatic heterocycle) in the present invention is meant thienyl, furanyl, thiazolyl, imidazolyl, (is)oxazolyl, pyridyl, pyrimidinyl, (iso)quinolinyl, naphthyridinyl, benzimidazolyl, and benzoxazolyl. The heterocycle is unsubstituted or substituted by 1 to 3 substituents selected from alkyl, O-alkyl and S-alkyl, OH, SH, -CN, halogen, 1,3-dioxolanyl, $CF_3$, $NO_2$, $NH_2$, $NHCH_3$, $N(CH_3)_2$, NHCO-alkyl, $-(CH_2)_mCO_2H$, $-(CH_2)_mCO_2$-alkyl, $-(CH_2)_mSO_3H$, -NH alkyl, $-N(alkyl)_2$, $-CH_2)_mPO_3H_2$, $-(CH_2)_mPO_3(alkyl)_2$, $-(CH_2)_mSO_2NH_2$, and $-(CH_2)_mSO_2NH$-alkyl wherein alkyl is defined as above and m is 0, 1, 2, or 3. A preferable heteroaryl group of the present invention is 2-, 3- or 4-pyridine. Examples of substituted heteroaryl groups include 2-chloropyridin-4-yl, 6-methoxynaphthyridin-2-yl, 6-trifluoromethylpyrimidin-2-yl, 5,6-diethoxybenzimidazol-2-yl, and 4-chloro-5-nitro-7-acetamidobenzoxazol-2-yl.
The symbol "-" means a covalent bond.
The term "pharmaceutically acceptable salt, ester and amide as used herein refers to those carboxylate salts,

amino acid addition salts, esters, and amides of the compounds of the present invention which are, within the scope of sound medical judgement, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively nontoxic, inorganic and organic acid addition salts of compounds of the present invention. These salts can be prepared in situ during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laureate, borate, benzoate, lactate, phosphate, tosylate, citrate, maleate, fumarate, succinate, tartrate, naphthylate mesylate, glucoheptonate, lactobionate and laurylsulphonate salts, and the like. These may include cations based on the alkali and alkaline earth metals, such as sodium, lithium, potassium, calcium, magnesium, and the like, as well as, nontoxic ammonium, quaternary ammonium, and amine cations including, but not limited to ammonium, tetramethylammonium, tetraethylammonium, methylamine, dimethylamine, trimethylamine, triethylamine, ethylamine, and the like. (See, for example, Berge S.M., et al., Pharmaceutical Salts, *J. Pharm. Sci.*, 1977;66:1-19)

Examples of pharmaceutically acceptable, nontoxic esters of the compounds of this invention include $C_1$-$C_6$ alkyl esters wherein the alkyl group is a straight or branched chain. Acceptable esters also include $C_5$-$C_7$ cycloalkyl esters as well as arylalkyl esters such as, but not limited to benzyl. $C_1$-$C_4$ alkyl esters are preferred. Esters of the compounds of the present invention may be prepared according to conventional methods.

Examples of pharmaceutically acceptable, nontoxic amides of the compounds of this invention include amides derived from ammonia, primary $C_1$-$C_6$ alkyl amines and secondary $C_1$-$C_6$ dialkyl amines wherein the alkyl groups are straight or branched chain. In the case of secondary amines, the amine may also be in the form of a 5- or 6-membered heterocycle containing one nitrogen atom. Amides derived from ammonia, $C_1$-$C_3$ alkyl primary amides and $C_1$-$C_2$ dialkyl secondary amides are preferred. Amides of the compounds of the invention may be prepared according to conventional methods.

All of the foregoing pharmaceutically acceptable salts, ester and amides are readily prepared and used by pharmaceutical scientists and medical personnel to whom this invention is directed. Such compounds are those that are pharmaceutically acceptable and can be administered to animals, including humans, for the treatments and other uses described herein.

In addition, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention.

Certain of the compounds of the present invention possess one or more chiral centers and each center may exist in the R or S configuration. The present invention includes all diastereomeric, enantiomeric, and epimeric forms as well as the appropriate mixtures thereof. Additionally, the compounds of the present invention may exist as geometric isomers. The present invention includes all cis, trans, syn, anti, entgegen (E), and zusammen (Z) isomers as well as the appropriate mixtures thereof .

Representative compounds of the invention are shown below in Table 1.

TABLE 1

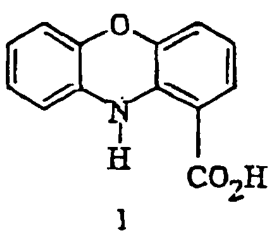

1

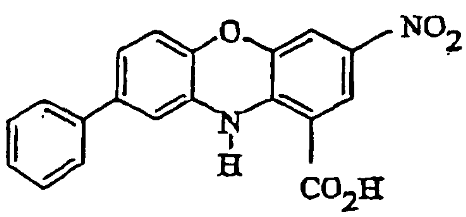

2

TABLE 1 (continued)

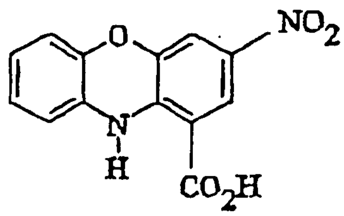

3

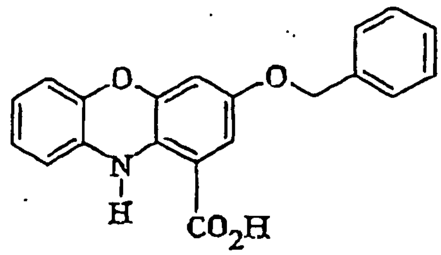

4

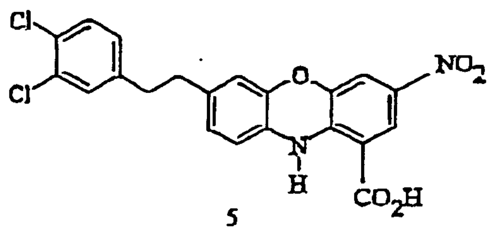

5

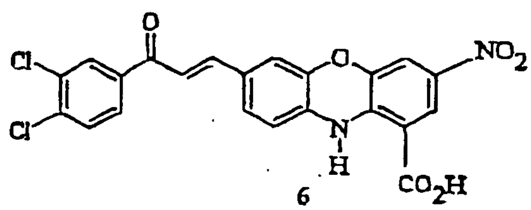

6

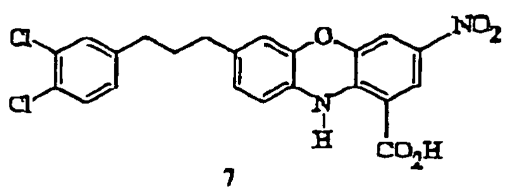

7

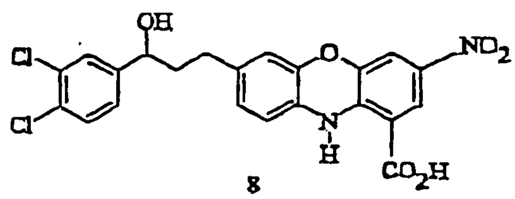

8

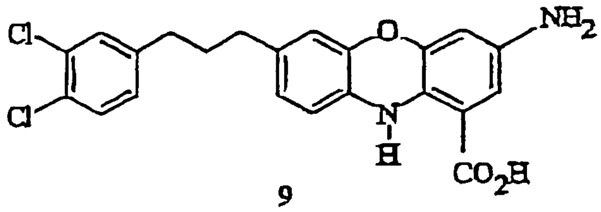

9

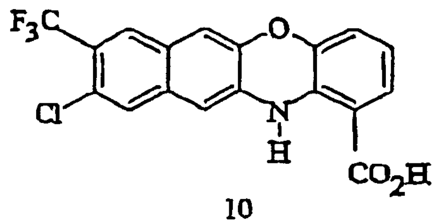

10

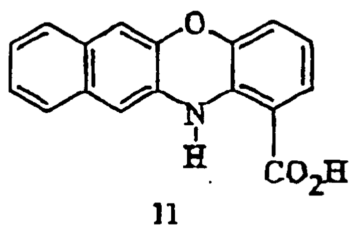

11

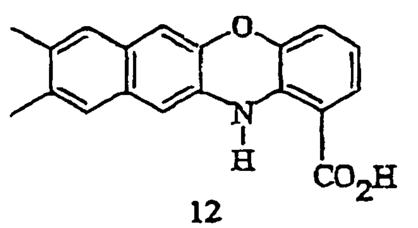

12

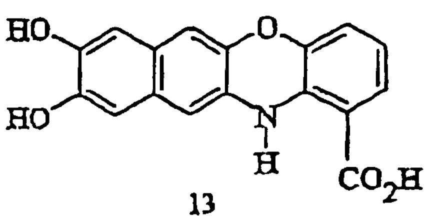

13

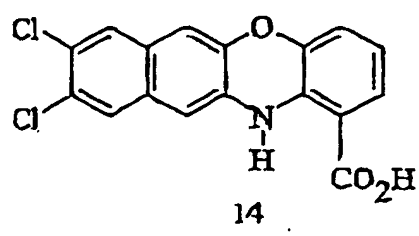

14

TABLE 1 (continued)

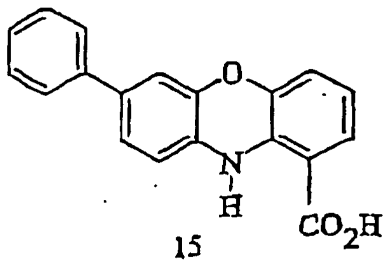

15

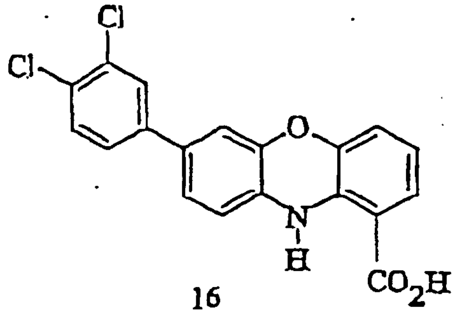

16

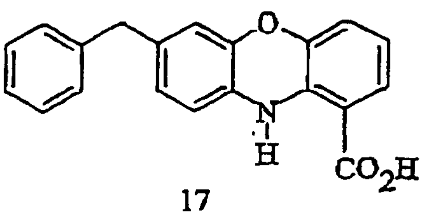

17

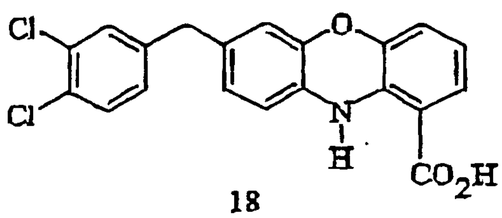

18

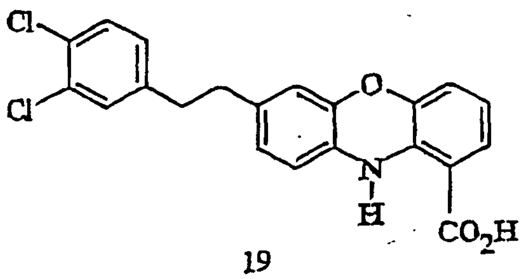

19

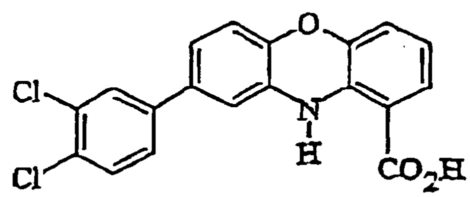

20

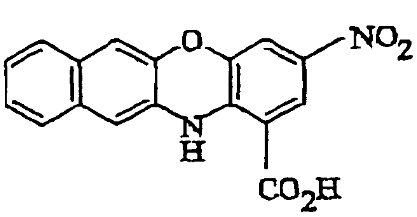

21

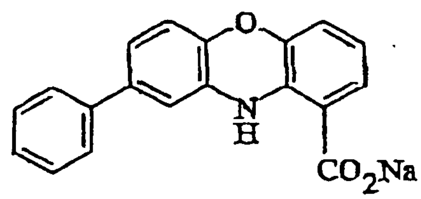

22

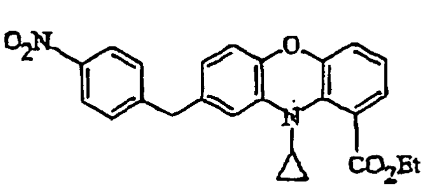

23

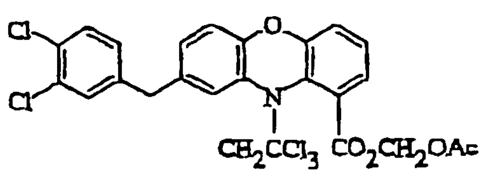

24

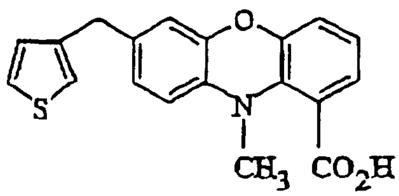

25

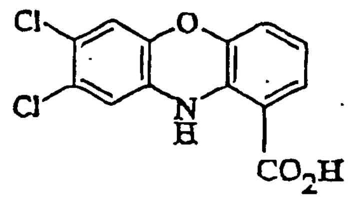

26

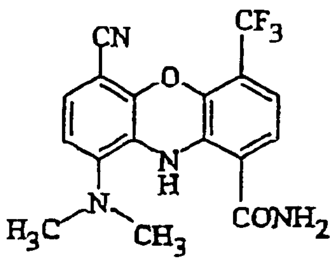

27

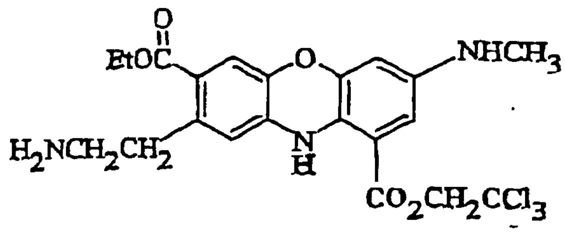

28

Representative compounds of the present invention, which are encompassed by Formula I include, but are not limited to the compounds in Table 1 and their pharmaceutically acceptable acid or base addition salts, the ester or amides thereof.

The invention compounds of Formula I are readily prepared from commercially available reactants, utilizing synthetic methodologies well-known and routinely used by those skilled in the art of synthetic organic chemistry. While the invention compounds can be prepared by any number of alternative processes, typical synthetic routes utilized to prepare illustrative invention compounds are presented in Schemes 1 to 4. In the Schemes, $R^2$, $R^5$, and $R^6$ have the meanings defined above for Formula I.

Scheme 1

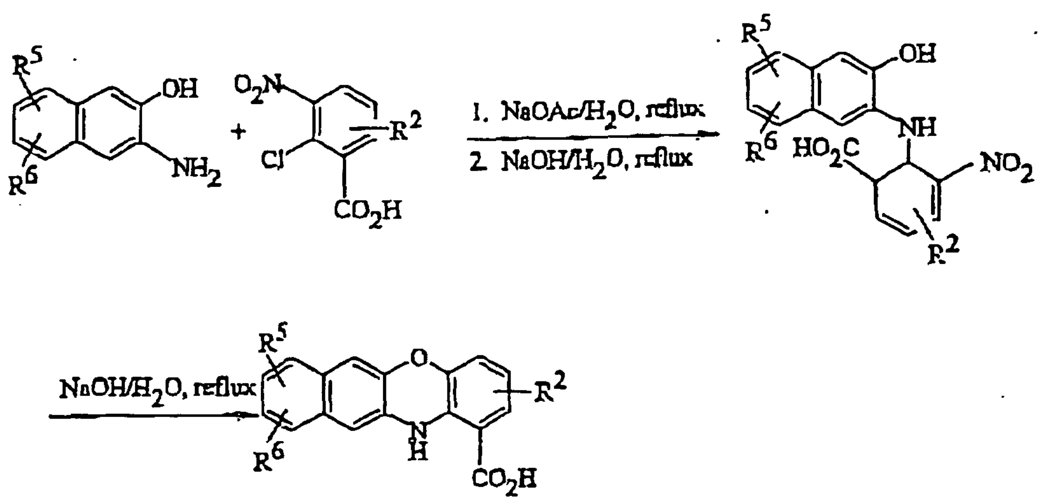

Scheme 2

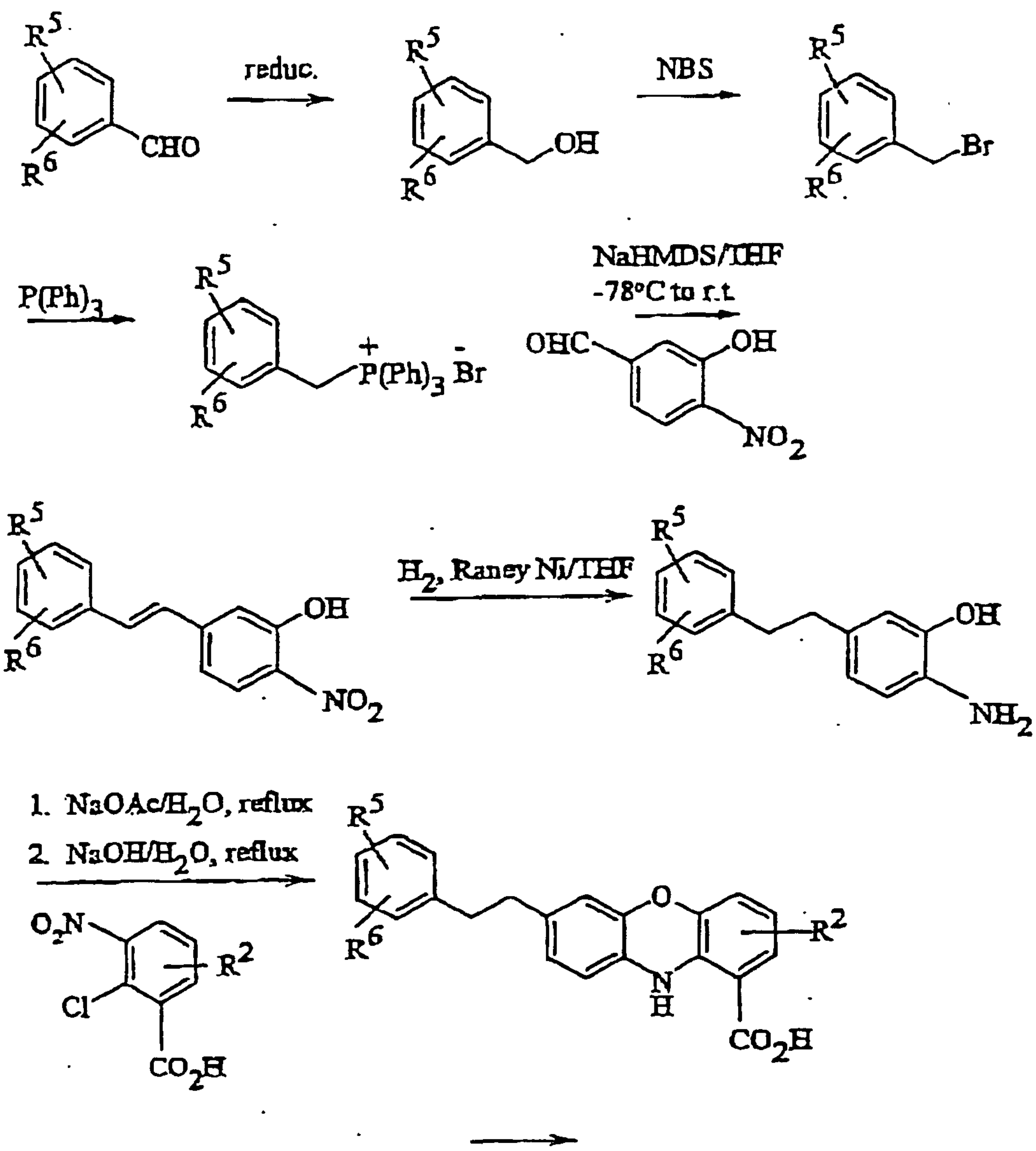
reduc.
NBS
P(Ph)3
NaHMDS/THF
-78°C to r.t.
H2, Raney Ni/THF
1. NaOAc/H2O, reflux
2. NaOH/H2O, reflux

Scheme 3

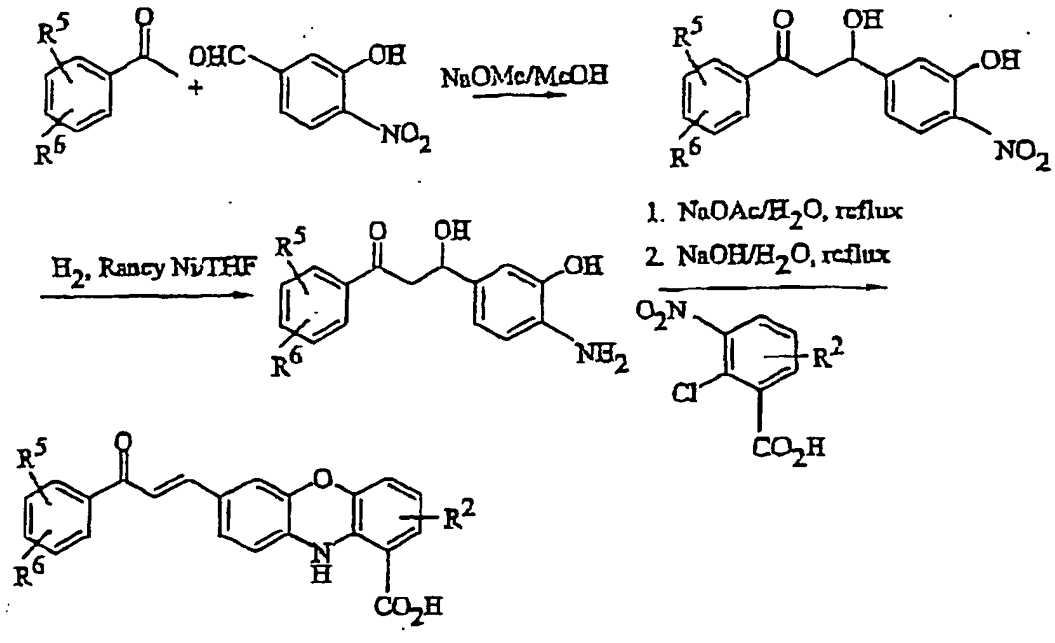
NaOMe/MeOH
H2, Raney Ni/THF
1. NaOAc/H2O, reflux
2. NaOH/H2O, reflux

Scheme 4

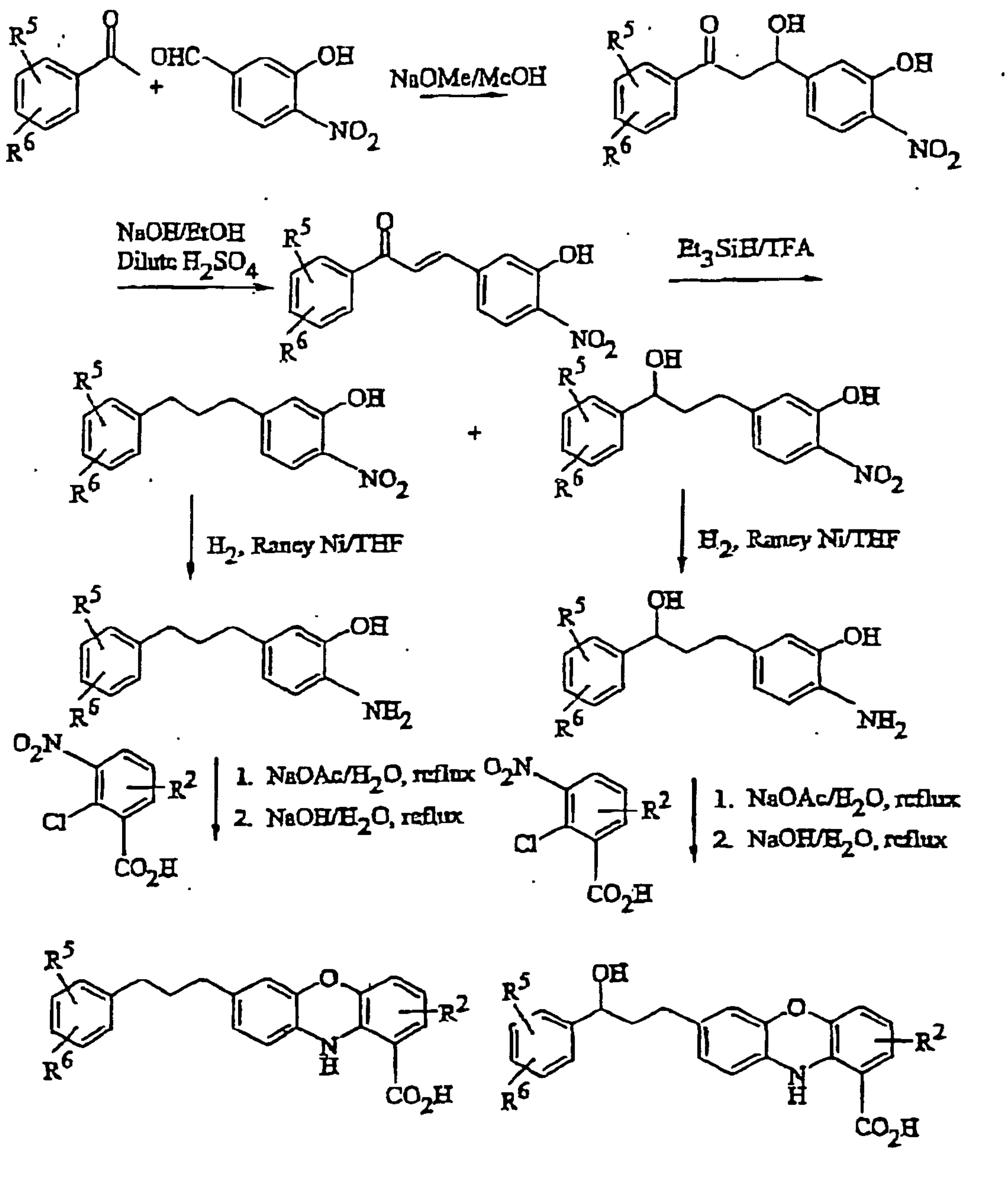


Scheme 1 involves the coupling of a 3-aminonaphthalen-2-ol with a 2-chloro-3-nitrobenzoic acid to form the corresponding secondary amine, which is subsequently cyclized under basic conditions to form the desired benzo[b] phenoxazinecarboxylic acid.

According to Scheme 2, a benzaldehyde is reduced to the corresponding benzyl alcohol (e.g., via a metal hydride) which is then converted to the benzyl bromide (e.g., N-bromosuccinimide). The benzyl bromide can be converted to the phosphonium salt which in turn is converted to the corresponding ylide upon treatment with a strong base, and the ylide is then treated with the desired 3-hydroxy-4-nitrobenzaldehyde to afford a 5-(2-phenylvinyl)-2-nitrophenol (i.e., Wittig reaction). The 5-(2-phenylvinyl)-2-nitrophenol is reduced to the 2-amino-5-(2-phenylethyl)phenol which is subsequently coupled to a 2-chloro-3-nitrobenzoic acid and cyclized to form the desired 7-(2-phenylethyl)phenoxazinecarboxylic acid.

Scheme 3 illustrates the formation of various 7-(3-oxo-3-phenylprop-1-enyl)phenoxazinecarboxylic acids. Initially, an acetophenone is coupled with a 3-hydroxy-4-nitrobenzaldehyde to afford the 3-hydroxy-3-(3-hydroxy-4-nitrophenyl)-1-phenylpropan-1-one. This nitro compound is reduced to the amine and the amine is coupled and cyclized (dehydration also results) as above for Scheme 2 to produce the 7-(3-oxo-3-phenylprop-1-enyl)phenoxazinecarboxylic

acid.

Finally, Scheme 4 shows the dehydration of a 3-hydroxy-3-(3-hydroxy-4-nitrophenyl)-1-phenylpropan-1-one to the enone, which is subsequently reduced to produce two products, the 2-nitro-5-(3-phenylpropyl)phenol and the 5-(3-hydroxy-3-phenylpropyl)-2-nitrophenol. Each compound can independently be reduced to the amine and coupled and cyclized as above to afford the final products 7-(3-phenylpropyl)phenoxazinecarboxylic add and 7-(3-hydroxy-3-phenylpropyl)phenoxazinecarboxylic acid, respectively.

The invention also includes radiolabeled compounds of Formula I that are useful for detecting and quantitating amyloid protein deposits. Such radiolabeled compounds are synthesized by standard methods, for example, by using a radiolabeled starting material in any of the foregoing schemes. Typical starting materials are those having a $^{13}C$, $^{19}F$, $^{15}N$, $^{11}C$, or other radioactive atom as part of the molecule.

In the first step of the present method of imaging amyloid deposits, a labeled compound of Formula I is introduced into a tissue or a patient in a detectable quantity. The compound is typically part of a pharmaceutical composition and is administered to the tissue or the patient by methods well known to those skilled in the art.

Those skilled in the art are familiar with the various ways to detect labeled compounds. For example, MRI, positron emission tomography (PET), or single photon emission computed tomography (SPECT) can be used to detect radiolabeled compounds. The label that is introduced into the compound will depend on the detection method desired. For example, if PET is selected as a detection method, the compound must possess a positron-emitting atom, such as $^{11}C$ or $^{18}F$.

Another example of a suitable label in a compound of Formula I is an atom such as $^{13}C$, $^{15}N$, or $^{19}F$ which can be detected using MRI, which is also sometimes called nuclear magnetic resonance (NMR). In addition, the labeled compounds of Formula I may also be detected by MRI using paramagnetic contrast agents.

Another example of detection is electron paramagnetic resonance (EPR). In this case, EPR probes which are well-known in the art, such as nitroxides, can be used.

The imaging of amyloid deposits can also be carried out quantitatively so that the amount of amyloid deposits can be determined.

In the methods of treating disorders related to amyloidosis according to the present invention, disorders such as Alzheimer's disease, a compound of Formula I can be administered either orally, rectally, parenterally (intravenously, intramuscularly, or subcutaneously), intracisternally, intravaginally, intraperitoneally, intravesically, locally (powders, ointments, or drops), or as a buccal or nasal spray. The invention provides pharmaceutical compositions comprising a compound of Formula I mixed with a carrier, diluent, or excipient.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles include water, ethanol, polyols (propyleneglycol, polyethyleneglycol, glycerol, and the like), suitable mixtures thereof, vegetable oils (such as olive oil), and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate, or (a) fillers or extenders, as for example, starches, lactose, sucrose, glucose, mannitol, and silicic acid; (b) binders, as for example, carboxymethylcellulose, alginates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example, paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft- and hard-filled gelatin capsules using such excipients as lactose or milk sugar, as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in

a delayed manner. Examples of embedding compositions which can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil, and sesame oil, glycerol, tetrahydrofurfuryl alcohol, polyethyleneglycols, and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar, and tragacanth, or mixtures of these substances, and the like.

Compositions for rectal administrations are preferably suppositories which can be prepared by mixing the compounds of the present invention with suitable nonirritating excipients or carriers such as cocoa butter, polyethyleneglycol or a suppository wax, which are solid at ordinary temperatures but liquid at body temperature and therefore, melt in the rectum or vaginal cavity and release the active component.

Dosage forms for topical administration of a compound of this invention include ointments, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

In a preferred embodiment of the invention, the compound is labeled and introduced into a patient in a detectable quantity and after sufficient time has passed for the compound to become associated with amyloid deposits, the labeled compound is detected noninvasively inside the patient. In another embodiment of the invention, a labeled compound of Formula I is introduced into a patient, sufficient time is allowed for the compound to become associated with amyloid deposits, and then a sample of tissue from the patient is removed and the labeled compound in the tissue is detected apart from the patient. In a third embodiment of the invention, a tissue sample is removed from a patient and a labeled compound of Formula I is introduced into the tissue sample. After a sufficient amount of time for the compound to become bound to amyloid deposits, the compound is detected.

The administration of the labeled compound to a patient can be by a general or local administration route. For example, the labeled compound may be administered to the patient such that it is delivered throughout the body. Alternatively, the labeled compound can be administered to a specific organ or tissue of interest. For example, it is desirable to locate and quantitate amyloid deposits in the brain in order to diagnose or track the progress of Alzheimer's disease in a patient.

The term "tissue" means a part of a patient's body. Examples of tissues include the brain, heart, liver, blood vessels, and arteries. A detectable quantity is a quantity of labeled compound necessary to be detected by the detection method chosen. The amount of a labeled compound to be introduced into a patient in order to provide for detection can readily be determined by those skilled in the art. For example, increasing amounts of the labeled compound can be given to a patient until the compound is detected by the detection method of choice. A label is introduced into the compounds to provide for detection of the compounds.

The term "patient" means humans and other animals such as horses, dogs, cats, and sheep. Those skilled in the art are also familiar with determining the amount of time sufficient for a compound to become associated with amyloid deposits. The amount of time necessary can easily be determined by introducing a detectable amount of a labeled compound of Formula I into a patient and then detecting the labeled compound at various times after administration.

The term "associated" means a chemical interaction between the labeled compound and the amyloid deposit. Examples of associations include covalent bonds, ionic bonds, hydrophilic-hydrophilic interactions, hydrophobic-hydrophobic interactions, and complexes.

The present invention also provides a method of inhibiting the aggregation of amyloid proteins to form amyloid deposits, by administering to a patient in need of inhibition of the aggregation of amyloid protein an amyloid protein inhibiting amount of a compound of Formula I. Those skilled in the art are readily able to determine an amyloid inhibiting amount by simply administering a compound of Formula I to a patient in increasing amounts until the growth of amyloid deposits is decreased or stopped. The rate of growth can be assessed using imaging or by taking a tissue sample from a patient and observing the amyloid deposits therein.

A patient in need of inhibition of the aggregation of amyloid proteins is a patient having a disease or condition in which amyloid proteins aggregate. Examples of such diseases and conditions include Mediterranean fever, Muckle-Wells syndrome, idiopathetic myeloma, amyloid polyneuropathy, amyloid cardiomyopathy, systemic senile amyloidosis, amyloid polyneuropathy, hereditary cerebral hemorrhage with amyloidosis, Alzheimer's disease, Downs syndrome, sickle cell anemia, scrapie, Parkinson's disease, Creutzfeldt-Jakob disease, kuru, Gerstmann-Sträussler-Scheinker syndrome, medullary carcinoma of the thyroid, isolated atrial amyloid, $\beta_2$-microglobulin amyloid in dialysis patients, inclusion body myositis, $\beta_2$-amyloid deposits in muscle wasting disease, and Islets of Langerhans diabetes type 2 insulinoma.

The compounds of the present invention can be administered to a patient at dosage levels in the range of about 0.1 mg/day to about 1000 mg/day. For a normal human adult having a body weight of about 70 kg, a dosage in the range of about 0.01 mg/kg to about 100 mg/kg of body weight per day is sufficient. The specific dosage used, however, can vary. For example, the dosage can depend on a number of factors including the requirements of the patient, the severity of the condition being treated, and the pharmacological activity of the compound being used. The determination of optimum dosages for a particular patient is well-known to those skilled in the art.

Armed with the disclosure provided herein (particularly the schemes and the synthetic examples described above) and knowledge common to all who practice in the field, those of ordinary skill in the art will be able to make and use the entire scope of compounds disclosed herein.

The invention is illustrated further by the following detailed examples which are not to be construed as limiting the invention in scope to the specific procedures described in them.

The starting materials and various intermediates may be obtained from commercial sources, prepared from commercially available organic compounds, or prepared using well-known synthetic methods.

Representative examples of methods for preparing intermediates of the invention are set forth in the examples below.

EXAMPLE 1

Synthesis of 3-nitrobenzo[b]phenoxazinecarboxylic acid (**Compound 21**)

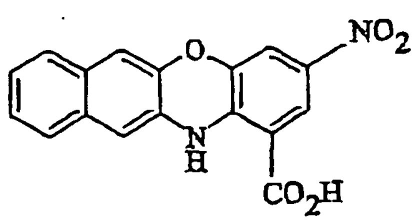

**1. 2-[(3-Hydroxy(2-naphthyl))amino]-3,5-dinitrobenzoic acid**

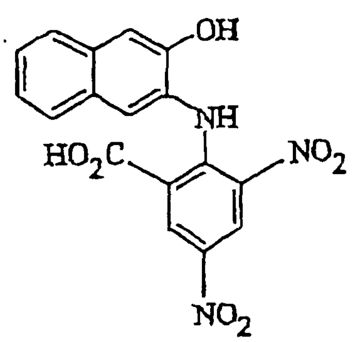

A mixture of 3-aminonaphthalen-2-ol (4.65 g, 0.029 mol), 2-chloro-3,5-dinitrobenzoic acid (7.20 g, 0.029 mol), water (30 mL), and 2N sodium acetate (NaOAc) (15 mL) is stirred and heated under reflux for 15 minutes. To the resulting thick paste is added 2N sodium hydroxide (NaOH) (15 mL), and the mixture is stirred and heated for another 15 minutes. The dark purple sodium salt is washed with cold brine. An aqueous solution of the filtrate is acidified with

dilute HCl and the free acid precipitates as a dark colored solid. The precipitate is triturated in boiling 10% MeOH/$H_2O$, filtered, rinsed with cold 10% MeOH/$H_2O$, and dried at room temperature in a vacuum oven overnight to yield an orange solid (9.00 g, 0.024 mol, 84%) as the desired product; mp 154-156°C.

| Analysis for $C_{17}H_{11}N_3O_7 \cdot 0.75\ H_2O$: | |
|---|---|
| Calcd | C 53.34; H 3.29; N 10.98. |
| Found | C 53.00; H 2.94; N 10.58. |

**2. 3-Nitrobenzo[b]phenoxazinecarboxylic acid**

A mixture of 2-[(3-hydroxy(2-naphthyl))amino]-3,5-dinitrobenzoic acid (8.50 g, 0.023 mmol), water (30 mL), and 2N sodium hydroxide (20 mL) is stirred and heated under reflux overnight. The resulting dark purple sodium salt is washed with cold brine and filtered. An aqueous solution of the salt is acidified with dilute HCl and the free acid precipitates as a dark colored solid. The filtrate is acidified with dilute HCl and the free acid precipitates as a dark colored solid. The precipitate is triturated in boiling 10% MeOH/$H_2O$, filtered, rinsed with cold 10% MeOH/$H_2O$, and dried at room temperature in a vacuum oven overnight to yield a brown solid (0.68 g, 0.002 mol, 7%) as the desired product (**compound 21**);
mp >230°C.

| Analysis for $C_{17}H_{10}N_2O_5 \cdot 0.29\ H_2O$: | |
|---|---|
| Calcd | C 62.35; H 3.26; N 8.55. |
| Found | C 61.96; H 2.95; N 8.96. |

EXAMPLE 2

Synthesis of 3-nitro-8-phenylphenoxazinecarboxylic acid (**Compound 2**)

**1. 2-[(2-Hydroxy-5-phenylphenyl)amino]-3,5-dinitrobenzoic acid**

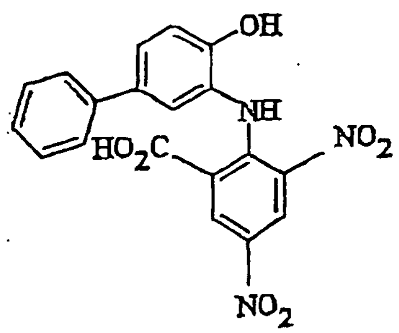

The title compound is prepared from 2-amino-4-phenylphenol (5.59 g, 0.03 mol), 2-chloro-3,5-dinitrobenzoic acid (7.45 g, 0.03 mol), water (30 mL), 2N NaOAc (15 mL), and 2N NaOH (15 mL) using the procedure of Example 1, Step 1 as a red solid (9.37 g, 0.023 mol, 78%); mp 215-217°C.

| Analysis for $C_{19}H_{13}N_3O_7 \cdot 1.13\ H_2O$: | |
|---|---|
| Calcd | C 54.90; H 3.70; N 10.11. |
| Found | C 54.51; H 3.57; N 9.84. |

**2. 3-Nitro-8-phenylphenoxazinecarboxylic acid**

2-[(2-Hydroxy-5-phenylphenyl)amino]-3,5-dinitrobenzoic acid (8.82 g, 0.022 mmol), water (50 mL), and 10N

NaOH (10 mL) are stirred and heated under reflux overnight. The reaction is worked up as in Example 1, Step 2, to afford the final product (**compound 2**) as a brown solid (5.75 g, 0.017 mol, 75%);
mp >250°C.

| Analysis for $C_{19}H_{12}N_2O_5 \cdot 0.10\ H_2O$: | |
|---|---|
| Calcd | C 65.18; H 3.51; N 8.00. |
| Found | C 64.79; H 3.30; N 8.18. |

EXAMPLE 3

Synthesis of 7-[2-(3,4-dichlorophenyl)ethyl]-3-nitrophenoxazinecarboxylic acid (**Compound 5**)

**1. Bromo[(3,4-dichlorophenyl)methyl]triphenylphosphorane**

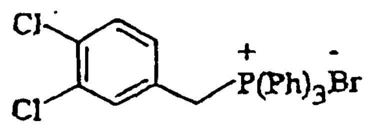

A mixture of 4-bromomethyl-1,2-dichlorobenzene (2.40 g, 0.01 mol), and triphenylphosphine (5.24 g, 0.02 mol) in toluene (30 mL) is stirred for 16 hours at room temperature. The solid is filtered, rinsed with toluene, and oven dried to yield a white powder (3.95 g, 0.0078 mol, 78%) as the desired product. $^1H$ NMR (δ ppm): 7.89-7.61 (m, 15H), 7.50 (d, J=2.3 Hz, 1H), 6.97 (dt, J=8.3 Hz, 2.3 Hz, 1H), 5.20 (d, J=15.9 Hz, 2H).

**2. 5-[2-(3,4-Dichlorophenyl)vinyl]-2-nitrophenol**

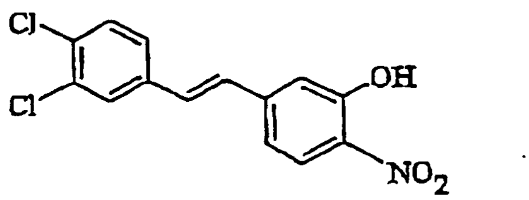

A solution of bromo[(3,4-dichlorophenyl)methyl]triphenylphosphorane (11.54 g, 22.98 mmol) in dry THF (250 mL) is cooled to -78°C. Sodium hexamethyldisilazane (NaHDMS) (1.0 M/THF, 50.33 mL, 50.33 mol) is added dropwise to maintain the temperature at -78°C. After stirring for 20 minutes, a solution of 3-hydroxy-4-nitrobenzaldehyde (4.22 g, 25.28 mmol) in THF (50 mL) is added dropwise. The resulting mixture is allowed to warm to room temperature within 3 hours. The mixture is then quenched with saturated ammonium chloride ($NH_4Cl$) and extracted with ethyl acetate (EtOAc). The organic layers are then washed with 0.1N HCl solution, $H_2O$, and brine, respectively. The solution is dried over sodium sulfate ($Na_2SO_4$) and concentrated in vacuo to give a light brown oil. Purification by flash chromatography (silica gel, 15% EtOAc/hexane) yields 4.29 g (13.8 mmol, 60%) of the desired product; mp 105-106°C.

| Analysis for $C_{14}H_9N_1O_3Cl_2 \cdot 0.08$ EtOAc: | |
|---|---|
| Calcd | C 54.23; H 3.06; N 4.42. |
| Found | C 54.33; H 3.04; N 4.02. |

**3. 2-Amino-5-[2-(3,4-dichlorophenyl)ethyl]phenol**

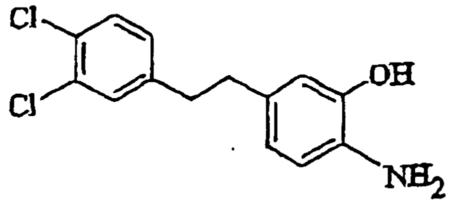

A sample of 5-[2-(3,4-dichlorophenyl)vinyl]-2-nitrophenol (4.17 g, 13.45 mmol) in THF (100 mL) is reduced in the presence of Raney Nickel (1 g) at 22°C to 29°C (ΔP = 4.3 psi). The reaction mixture is filtered, and the filtrate is concentrated in vacuo to give a brown solid, 3.5 g (12.4 mmol, 92%) of the desired product; mp 147-149°C.

| Analysis for $C_{14}H_{13}N_1O_1Cl_2$: | |
|---|---|
| Calcd | C 59.59; H 4.64; N 4.96. |
| Found | C 56.98; H 4.60; N 4.71. |

**4. 7-[2-(3,4-Dichlorophenyl)ethyl]-3-nitrophenoxazinecarboxylic acid**

A mixture of 2-amino-5-[2-(3,4-dichlorophenyl)ethyl]phenol (0.73 g, 2.59 mmol), 2-chloro-3,5-dinitrobenzoic acid (0.68 g, 2.59 mmol), water (3 mL), and 2N sodium acetate (1.3 mL) is stirred and heated under reflux for 5 hours. To the resulting thick paste is added 2N sodium hydroxide (15 mL) and the mixture is stirred and heated for another 3 hours. The reaction mixture is acidified with dilute HCl and the free acid forms as a dark precipitate. The precipitate is filtered off and washed with $H_2O$. Purification by flash chromatography (silica gel, toluene:EtOAc:AcOH [30:5:1]) yields 0.21 g (0.47 mmol, 18%) of the desired product (compound 5); mp >250°C.

| Analysis for $C_{21}H_{14}N_2O_5Cl_2 \cdot 0.3$ toluene-0.25 $H_2O$: | |
|---|---|
| Calcd | C 58.12; H 3.57; N 5.87. |
| Found | C 57.77; H 3.29; N 5.52. |

EXAMPLE 4

Synthesis of 7-[3-(3,4-Dichlorophenyl)-3-oxoprop-1-enyl]-3-nitrophenoxazinecarboxylic acid (**Compound 6**)

**1. 1-(3,4-Dichlorophenyl)-3-hydroxy-3-(3-hydroxy-4-nitrophenyl)propan-1-one**

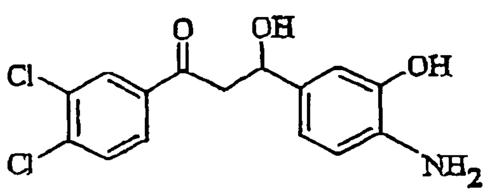

Sodium hydroxide (4.88 g, 0.122 nimol) is dissolved in water (80 mL) and 95% EtOH (80 mL) and cooled to 0°C with an ice-$H_2O$ bath.
1-(3,4-Dichlorophenyl)ethanone (23.10 g, 0.122 mol) is added in one portion. After the addition, the mixture is warmed to 15°C. 3-hydroxy-4-nitrobenzaldehyde (20.42 g, 0.122 mol) is then added with rigorous stirring. After stirring for 5 minutes, the reaction mixture is diluted with 95% EtOH (300 mL). The resulting tan mixture is stirred at room temperature overnight. The reaction mixture is acidified with 3N HCl and stirred for 30 minutes. The resulting yellow solid is filtered

off, washed with $H_2O$, washed with 5% $MeOH/CH_2Cl_2$, and oven dried (40°C) to yield a yellow solid (25.00 g, 0.074 mol, 61%) of the title compound; mp 163-166°C.

| Analysis for $C_{15}H_9Cl_2NO_5$: | |
|---|---|
| Calcd | C 50.59; H 3.11; N 3.93. |
| Found | C 50.61; H 2.81; N 3.81. |

**2. 2-Amino-5-[3-(3,4-dichlorophenyl)-3-hydroxypropyl]phenol**

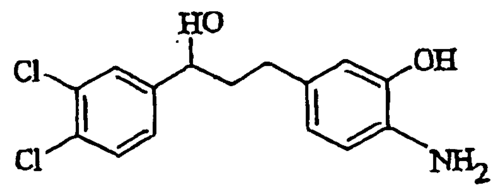

The title compound is prepared from 1-(3,4-dichlorophenyl)-3-hydroxy-3-(3-hydroxy-4-nitrophenyl)propan-1-one (6.5 g, 18.25 mmol) in the presence of Raney Nickel (4.0 g) in THF (100 mL) at 25°C (ΔP = 6.3 psi) using the procedure described in Example 5, Step 3. The reaction mixture is concentrated in vacuo and dried in an oven vacuum (30°C) to give a brown solid (5.9 g, 18.08 mmol, 99%) of the desired product. MS: 326.0 ($M^+$). $^1H$ NMR (δ ppm): 9.87 (s, 1H), 8.08 (d, J=2 Hz, 1H), 7.87 (dd, J=8.3 Hz, 1.7 Hz, 1H), 7.76 (d, J=8.3 Hz, 1H), 6.67 (s, 1H), 6.53 (d, J=8.1 Hz, 1H), 6.48 (d, J=8.0 Hz, 1H), 5.08 (d, J=4.2 Hz, 1H), 4.82 (ddd, J=4.2, 4.2, 8.4 Hz, 1H), 4.38 (s, 2H), 3.32 (dd, J=8.8, 16.6 Hz, 1H), 3.02 (dd, J=4.4, 15.4 Hz, 1H).

**3. 7-[3-(3,4-Dichlorophenyl)-3-oxoprop-1-enyl]-3-nitrophenoxazinecarboxylic acid**

The title compound is prepared from 2-amino-5-[3-(3,4-dichlorophenyl)-3-hydroxypropyl]phenol (2.00 g, 6.13 mmol), 2-chloro-3,5-dinitrobenzoic acid (1.51 g, 6.13 mmol), water (5 mL), 2N sodium acetate (3 mL), and 2N sodium hydroxide (3 mL) using the procedure described in Example 5, Step 4. This procedure yields a dark solid (0.5 g, 1.06 mmol, 17%) as the desired product (compound 6); mp >200°C.

| Analysis for $C_{22}H_{12}N_2O_6Cl_2 \cdot 0{\cdot}05\ H_2O$: | |
|---|---|
| Calcd | C 55.96; H 2.58; N 5.93. |
| Found | C 55.57; H 2.85; N 6.07. |

EXAMPLE 5

Synthesis of 7-[3-(3,4-Dichlorophenyl)propyl]-3-nitrophenoxazine carboxylic acid (**Compound 7**)

**1. 5-[3-(3,4-Dichlorophenyl)-3-hydroxyprop-1-enyl]-2-nitrophenol**

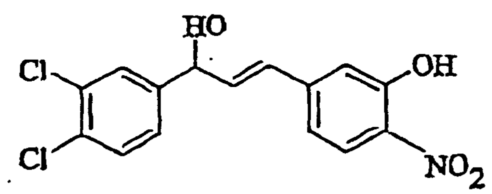

A solution of 1-(3,4-dichlorophenyl)-3-hydroxy-3-(3-hydroxy-4-nitrophenyl)propan-1-one (15.0 g, 42.12 mmol), 15% of NaOH (100 mL), and EtOH (200 mL) is heated to reflux for 30 minutes and then cooled to room temperature and allowed to stir overnight. The reaction mixture is acidified with 10% $H_2SO_4$ and stirred for 30 minutes. The precipitate is filtered off, washed with $H_2O$, then 95% EtOH, and oven dried (40°C) to yield a red solid (13.7 g, 40.51 mmol, 96%) of the title compound; mp 178-180°C.

| Analysis for $C_{15}H_9Cl_2NO_4 \cdot 0.4\ H_2O$: | |
|---|---|
| Calcd | C 52.17; H 2.86; N 4.06. |
| Found | C 51.78; H 2.62; N 3.68. |

**2. 5-[3-(3,4-Dichlorophenyl)propyl]-2-nitrophenol and 5-[3-(3,4-dichlorophenyl)-3-hydroxypropyl]-2-nitrophenol**

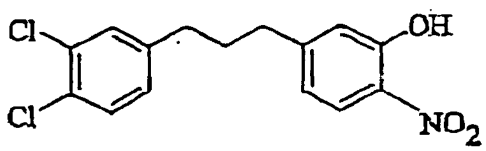

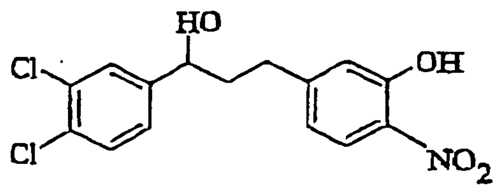

A solution of 5-[3-(3,4-dichlorophenyl)-3-hydroxyprop-1-enyl]-2-nitrophenol (5.97 g, 17.65 mmol), $Et_3SiH$ (8.2 g, 70.62 mmol) in TFA (70 mL) is stirred at room temperature for 10 days. The reaction mixture is concentrated to dryness, and the resulting residue is dissolved in $H_2O$ (100 mL) and extracted with EtOAc. The organic layer is dried ($MgSO_4$) and concentrated to give a brown oil. Purification by flash chromatography (silica gel, 1% MeOH/$CH_2Cl_2$) affords 3.8 g (11.65 mmol, 66%) of the desired product:
5-[3-(3,4-dichlorophenyl)propyl]-2-nitrophenol. MS: 321.9 (M+) and 2.0 g (5.8 mmol, 33%) of 5-[3-(3,4-dichlorophenyl)-3-hydroxypropyl]-2-nitrophenol; mp 90-93°C.

| Analysis for $C_{15}H_{15}N_1O_4Cl_2$: | |
|---|---|
| Calcd | C 58.34; H 4.39; N 4.07. |
| Found | C 52.38; H 3.97; N 3.91. |

**3. 2-Amino-5-[3-(3, 4-dichlorophenyl)propyl]phenol**

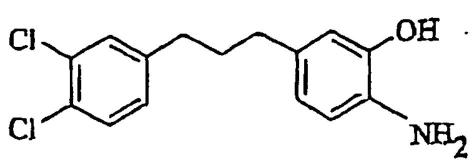

The title compound is prepared from 5-[3-(3, 4-dichlorophenyl)propyl]-2-nitrophenol (7.0 g, 21.46 mmol) and Raney Nickel (3.0 g) in THF (100 mL) at 24°C to 28°C (ΔP = 38.1 psi) using the procedure described in Example 5, Step 3. Purification by flash chromatography (silica gel, 20%, 50%, 80% EtOAc/Hexane) affords a white solid (1.16 g, 3.92 mmol, 18%) of the desired product; mp 79-82°C.

| HRMS analysis for $C_{15}H_{15}N_1O_1Cl_2$: | |
|---|---|
| Calcd | 296.0609. |
| Found | 296.0621 (Δ = 4.05 ppm). |

**4. 7-[3-(3,4-Dichlorophenyl)propyl]-3-nitrophenoxazine carboxylic acid**

The title compound is prepared from 2-amino-5-[3-(3,4-dichlorophenyl)propyl]phenol (1.11 g, 3.75 mmol), 2-chloro-3,5-dinitrobenzoic acid (0.93 g, 3.75 mmol), water (3 mL), 2N sodium acetate (2 mL), and 2N sodium hydroxide (2 mL) using the procedure described in Example 5, Step 4. This procedure yields a dark solid (1.50 g, 3.27 mmol, 87%) as the desired product (**compound 7**); mp 215-217°C.

| Analysis for $C_{22}H_{16}N_2O_5Cl_2 \cdot 0.45\ H_2O \cdot 0.05\ C_7H_3N_2O_6Cl$ (2-chloro-3,5-dinitrobenzoic acid): | |
|---|---|
| Calcd | C 55.96; H 3.58; N 6.13. |
| Found | C 55.60; H 3.20; N 5.88. |

EXAMPLE 6

Synthesis of 7-[3-(3,4-Dichlorophenyl)-3-hydroxypropyl]-3-nitrophenoxazine carboxylic acid (**Compound 8**)

**1. 2-Amino-5-[3-(3,4-dichlorophenyl)-3-hydroxypropyl]phenol**

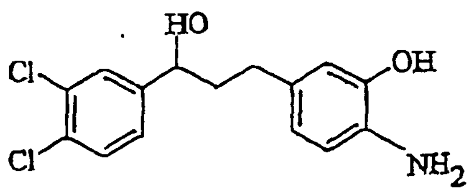

The title compound is prepared from 5-[3-(3,4-dichlorophenyl)-3-hydroxypropyl]-2-nitrophenol (2.0 g, 5.86 mmol) is Raney Nickel (0.5 g) in THF (50 mL) at 24°C to 25°C (ΔP = 38.3 psi) using the procedure described in Example 5, Step 3. This procedure yields an orange solid (1.43 g, 4.58 mmol, 79%) of the desired product; mp 121-123°C.

| Analysis for $C_{15}H_{15}N_1O_2Cl_2$: | |
|---|---|
| Calcd | C 57.71; H 4.84; N 4.49. |
| Found | C 57.40; H 4.71; N 4.35. |

**2. 7-[3-(3,4-Dichlorophenyl)-3-hydroxypropyl]-3-nitrophenoxazine carboxylic acid**

The title compound is prepared from 2-amino-5-[3,4-dichlorophenyl)-3-hydroxypropyl]phenol (2.39 g, 7.66 mmol), 2-chloro-3,5-dinitrobenzoic acid (1.89 g, 7.66 mmol), water (6 mL), 2N sodium acetate (4 mL), and 2N sodium hydroxide (4 mL) using the procedure described in Example 5, Step 4. This procedure affords a dark solid (3.40 g, 7.15 mmol, 93%) as the desired product (**compound 8**); mp 218-220°C.

| Analysis for $C_{22}H_{16}N_2O_6Cl_2 \cdot 0.75\ H_2O \cdot 0.05\ C_7H_3N_2O_6Cl$ (2-Chloro-3,5-dinitro-benzoic acid): | |
|---|---|
| Calcd | C 53.57; H 3.55; N 5.87. |
| Found | C 53.20; H 3.25; N 5.85. |

EXAMPLE 7

Synthesis of 3-Amino-7-[3-(3,4-dichlorophenyl)propyl]phenoxazine carboxylic acid (**Compound 9**)

The title compound is prepared from 7-[3-(3,4-dichlorophenyl)propyl]-3-nitrophenoxazinecarboxylic acid (**compound 8**) (0.86 g, 1.87 mmol) and Raney Nickel (0.2 g) in THF (50 mL) at 24°C to 26°C (ΔP = 13.3 psi) using the procedure described in Example 5, Step 3. This procedure yields a dark solid (0.13 g, 0.3 mmol, 16%) of the desired product (**compound 9**); mp 213-216°C.

| Analysis for $C_{22}H_{18}N_2O_3Cl_2 \cdot 0.55\ H_2O$: | |
|---|---|
| Calcd | C 60.16; H 4.38; N 6.38. |
| Found | C 59.77; H 4.20; N 6.03. |

EXAMPLE 8

The following compounds are prepared essentially according to the procedures described in Examples 1-7 and shown in Schemes 1-4:

(a) Phenoxazinecarboxylic acid (**compound 1**);
(b) 3-Nitrophenoxazinecarboxylic acid (**compound 3**);
(c) 3-(Phenylmethoxy)phenoxazinecarboxylic acid (**compound 4**);
(d) 9-Chloro-8-(trifluoromethyl)benzo[b]phenoxazinecarboxylic acid (**compound 10**);
(e) Benzo[b]phenoxazinecarboxylic acid (**compound 11**);
(f) 8,9-Dimethylbenzo[b]phenoxazinecarboxylic acid (**compound 12**);
(g) 8,9-Dihydroxybenzo[b]phenoxazinecarboxylic acid (**compound 13**);
(h) 8,9-Dichlorobenzo[b]phenoxazinecarboxylic acid (**compound 14**);
(i) 7-Phenylphenoxazinecarboxylic acid (**compound 15**);
(j) 7-(3,4-Dichlorophenyl)phenoxazinecarboxylic acid (**compound 16**);
(k) 7-Benzylphenoxazinecarboxylic acid (**compound 17**);
(l) 7-[(3,4-Dichlorophenyl)methyl]phenoxazinecarboxylic acid (**compound 18)**;
(m) 7-[2-(3,4-Dichlorophenyl)ethyl]phenoxazinecarboxylic acid **(compound 19)**; and
(n) 8-(3,4-Dichlorophenyl)phenoxazinecarboxylic acid (**compound 20**).

As noted above, the compounds of Formula I are useful because of their ability to inhibit amyloid protein aggregation. The inhibitory activity of the invention compounds has been determined in several biological assays routinely utilized by those skilled in the art to measure such amyloid inhibition. Representative invention compounds have been evaluated in the following amyloid assays.

**1. BASSR (Beta-Amyloid Self-Seeding Radioassay)**

An assay for inhibitors of self-seeded amyloid fibril growth

**Materials:**

Stock Solutions:

*Assay Buffer* - 50 mM sodium phosphate, pH 7.5, 100 mM NaCl, 0.02% $NaN_3$, 1 M urea (filter and store at 4°C)
*Soluble A*β *(1-40) peptide* (Bachem, Torrance, CA) - 22 mg/mL in deionized $H_2O$ (is stored in aliquots at -20°C; is kept on ice when thawed) will self-seed after 1 week storage. Typically, the solution is stored until no lag phase is seen in the assay.
$^{125}$*I-labeled A*β *(1-40)*-150K to 350K cpm/μL in 100% acetonitrile - 0.1% trifluoroacetic acid (TFA)-1% β-mercaptoethanol (aliquots stored at -20°C). $^{125}$I-labeled Aβ (1-40) is made in accordance with the procedure set forth by LeVine H., III, in *Neurobiol. Aging*, 1995;16:755, or this reagent may be purchased from Amersham, Arlington Heights, Illinois.
*Final assay conditions:* 30 μM soluble Aβ(1-40) in deionized water in assay buffer + 20K to 50K cpm $^{125}$I-labeled Aβ (1-40) per assay. Compound to be tested is dissolved in dimethylsulfoxide (DMSO), typically 5 to 50 mM stock, such that the final concentration of DMSO is <1% v/v in the assay.
**Assay:** Reaction mixture for 50 assays (on ice) is comprised of 0.1 to 0.2 μL of $^{125}$I-labeled A$^{125}$*I-labeled A*β(1-40) + 1 μL of soluble *A*β (1-40) + 13.5 μL assay buffer per assay. The following are the amounts of the components of the reaction mixture sufficient for 50 assay wells.

5-10 μL $^{125}$I-labeled *A*β(1-40) dried down
675 μL assay buffer
50 μL soluble *A*β(1-40)

Assay Method:

1) The reaction mixture of above is prepared by mixing components and storing on ice.
2) 14.5 µL of the reaction mixture is pipetted into each of 50 wells on a polypropylene U-bottom 96-well microtiter plate on ice (Costar 3794).
3) 1.7 µL of diluted compound to be tested is added to each well in a column of eight, including solvent control. Serial 3-fold dilutions from 1 mM (100 µM final) in assay buffer - urea = 7 dilutions + zero. Each 96-well plate can therefore accommodate 11 samples + 1 Congo Red control (0.039-5 µM final in 2-fold steps).
4) The plate with aluminum film (Beckman 53 8619) is sealed and incubated for 10 minutes on ice.
5) The temperature is raised to 37°C and incubated for 3 to 5 hours (depending on the lot of the peptide).
6) The aluminum film is removed and 200 µL/well of ice cold assay buffer with urea is added. The radiolabeled fibrils are collected by vacuum filtration through 0.2 µm pore size GVWP filters in 96-well plates (Millipore MAGV N22, Bedford, MA). The radioactivity of the filters is determined by using standard methods well-known to those skilled in the art.

**2. BASST (Beta-Amyloid Self-seeding, ThioflavinT)**

An assay for inhibitors of self-seeded amyloid fibril growth

**Materials:**

Stock Solutions:

*Assay Buffer*-50 mM sodium phosphate, pH 7.5, 100 mM $NaCl_2$ 0.02% $NaN_3$, 1 M urea (filter and store at 4°C)
*Soluble A*β *(1-40)*-2.2 mg/mL in deionized $H_2O$ (is stored in aliquots at -20°C; is kept on ice when thawed) will self-seed after 1 week storage. Typically, the solution is stored until no lag phase is seen in the assay.
*Final assay conditions:* 30 µM soluble *A*β(1-40) in deionized water in assay buffer. Compound to be tested is dissolved in DMSO, typically 5 to 50 mM stock, such that the final concentration of DMSO is <1% v/v in the assay.
**Assay**: Reaction mixture for 50 assays (on ice) is comprised of 1 µL of soluble *A*β (1-40) + 13.5 µL assay buffer per assay. The following are the amounts of the components of the reaction mixture that result in each of the 50 assay wells.

50 µL soluble *A*β(1-40)
675 µL assay buffer

Assay Method:

1) The reaction mix above is prepared by mixing the components and storing on ice.
2) 14.5 µL of reaction mixture is pipetted into each of 50 wells of a polystyrene U-bottom, 96-well microtiter plate (Corning 25881-96) on ice.
3) 1.7 µL of diluted compound to be tested is added to each well in a column of eight, including solvent control. Serial 3-fold dilutions from 1 mM (100 µM final) in assay buffer - urea = 7 dilutions + zero. Each 96-well plate can therefore accommodate 11 samples + 1 Congo Red control (0.039-5 µM final in 2-fold steps).
4) The plate with aluminum film is sealed and incubated for 10 minutes on ice.
5) The temperature is raised to 37°C and incubated for 3 to 5 hours (depending on the lot of the peptide).
6) The aluminum film is removed and 250 µL/well of 5 µM thioflavin T (ThT) [T-3516, Sigma-Aldrich] is added in 50 mM glycine-NaOH, pH 8.5. Fluorescence is read on a plate reader (ex = 440 nm/20 nm ; em = 485 nm/20 nm) within 5 minutes.

**3. BAPA (Beta-Amyloid Peptide Aggregation)**

This assay is used to provide a measure of inhibition by a compound against the aggregation behavior of the beta amyloid peptide.

The purpose of this assay is to provide a higher volume method of assaying the amount of beta amyloid aggregation using an endpoint assay based on filtration. In this assay, hexafluoroisopropanol (HFIP) is used to break down the initial amyloid peptide to a monomer state and a concentration of 33 μM is used, a concentration which is high enough so that aggregation will occur at pH 6.0 in several hours.

Method:

**β-Amyloid Peptide Aggregation, pH 6.0 (BAPA)**

To a 96 well plate (Costar 3794) is added 25 μL 50 mM Phosphate Buffer (pH 6.0), 10 μL 0.5 mg/mL Aβ (1-40) peptide in 20% HFIP + 0.1 μL/assay radioiodinated $^{125}$I Aβ (1-40) [$^{125}$I Aβ(1-40)], and 1 μL of the compound to be tested, starting at 50 mM with a concentration of DMSO <1%. The reaction is incubated for 2 to 4 hours at room temperature. The reaction is stopped with 200 μL of 50 mM phosphate buffer, pH 6.0, and filtered through a 0.2 μm 96-well filter plate (Millipore MAGU N22). The filter plate is washed with 100 μL of the same phosphate buffer. Aggregation is detected on a Microbeta counter after impregnating the filters with Meltilex (1450-441) and is corrected for background.

**4. BATYM ASSAY**

Methods:

Required Aβ (1-42) (California Peptide) is dried from its hexafluoroisopropanol (HFIP) stock solution. The Aβ (1-42) is dissolved in DMSO and then mixed with phosphate buffered saline (PBS) (pH 7.4). The mixed Aβ (1-42) solution is filtered with a GVWP 0.22 μm syringe filter (Millipore, Bedford, MA). The compound to be tested in DMSO (50 times concentrate) is put into each well (0.5 μL/well) of a 96-well plate. The Aβ (1-42) solution is added into each well (25 μL/well). The plate is centrifuged at 1000 g for 5 minutes and incubated at 37°C for 1 day (Aβ 1-42; final concentration 100 μM).

After incubation Thioflavin T (ThT) (30 μM) solution in glycine-NaOH buffer (pH 8.5, 50 mM) is added into each well (250 μL/well), fluorescence is measured (ex = 440/20 nm, em = 485/20 nm) using a fluorescence plate reader. The inhibitory activity is calculated as the reduction of fluorescence with the following formula:

$$\text{Inhibition (\%)} = \{(F(A\beta) - F(A\beta + \text{compound})\}/\{F(A\beta) - F(\text{solvent - compound})\} \times 100.$$

The $IC_{50}$s are calculated by a curve-fitting program using the equation given below. The data is obtained from two different experiments in triplicate.

$$F(x) = 100-100/\{1+(IC_{50}/10^x)^n\};$$

x = concentration of tested compound (M),
$IC_{50}$=(M),
n = Hill coefficient.

The results of these assays for compounds of the present invention are shown in Table 2.

TABLE 2

| Example No. | Compound No. | BASSR ($IC_{50}$ = μM) | BASST ($IC_{50}$ = μM) | BATYM ($IC_{50}$ = μM) | BAPA ($IC_{50}$ = μM) |
|---|---|---|---|---|---|
| 1 | 21 | 12, 11, 10 | 1 | 6.7 | 67 |
| 2 | 2 | 100, 60, 70, 60 | 1 | 4.15 | 5 |
| 3 | 5 | 60, >100(ppt) | 3, 1 | 7.89 (P) 3.02(Q) | 0.9 (Q) |
| 4 | 6 | 21, 22, 25 | 1 | 4.61 | >100 |

TABLE 2 (continued)

| Example No. | Compound No. | BASSR ($IC_{50}$ = μM) | BASST ($IC_{50}$ = μM) | BATYM ($IC_{50}$ = μM) | BAPA ($IC_{50}$ = μM) |
|---|---|---|---|---|---|
| 5 | 7 | 30 (ppt), 20, 6 | 0.5 | 1.88 | >100 |
| 6 | 8 | >100, 100, 100 | 0.6 | 4.18 | 100 |
| 7 | 9 | 60, >100 | 3 | 8.75 | 86 |
| 8b | 3 | 8 | 13, 40, 23, 23, 40, 13 | | 62, >100 (BAPA2) |
| 8c | 4 | 14 | 8.4, 45, 45, 8.44 | | 50, >50 (BAPA2) |
| ppt = Precipitate and indicates that a precipitate formed at the indicated concentration | | | | | |

The above data establishes that representative invention compounds are active in standard assays used to measure inhibition of protein aggregation. The compounds exhibit excellent specificity, for example, as shown in the BASST assay, as well as the BATYM assay. The compounds are thus useful to clinically inhibit amyloid protein aggregation and to image amyloid deposits for diagnostic use. The compounds will be used in the form of pharmaceutical formulations, and the following examples illustrate typical compositions.

EXAMPLE 9

| Tablet Formulation | |
|---|---|
| Ingredient | Amount |
| Compound of Example 1 | 50 mg |
| Lactose | 80 mg |
| Cornstarch (for mix) | 10 mg |
| Cornstarch (for paste) | 8 mg |
| Magnesium Stearate (1%) | 2 mg |
| | 150 mg |

The compound of Example 1 (Compound 21) is mixed with the lactose and cornstarch (for mix) and blended to uniformity to a powder. The cornstarch (for paste) is suspended in 6 mL of water and heated with stirring to form a paste. The paste is added to the mixed powder, and the mixture is granulated. The wet granules are passed through a No. 8 hard screen and dried at 50°C. The mixture is lubricated with 1% magnesium stearate and compressed into a tablet. The tablets are administered to a patient at the rate of 1 to 4 each day for prevention of amyloid protein aggregation and treatment of Alzheimer's disease.

EXAMPLE 10

**Parenteral Solution**

In a solution of 700 mL of propylene glycol and 200 mL of water for injection is added 20.0 g of Compound No. 7 (Example 5). The mixture is stirred and the pH is adjusted to 5.5 with hydrochloric acid. The volume is adjusted to 1000 mL with water for injection. The solution is sterilized, filled into 5.0 mL ampoules, each containing 2.0 mL (40 mg of Compound No. 7), and sealed under nitrogen. The solution is administered by injection to a patient suffering from medullary carcinoma of the thyroid and in need of treatment.

EXAMPLE 11

**Patch Formulation**

Ten milligrams of Compound No. 13 (Example 8g) is mixed with 1 mL of propylene glycol and 2 mg of acrylic-based polymer adhesive containing a resinous cross-linking agent. The mixture is applied to an impermeable backing ($30\ cm^2$) and applied to the upper back of a patient for sustained release treatment of amyloid polyneuropathy.

The invention and the manner and process of making and using it are now described in such full, clear, concise, and exact terms as to enable any person skilled in the art to which it pertains, to make and use the same. It is to be understood that the foregoing describes preferred embodiments of the present invention and that modifications may be made therein without departing from the scope of the present invention as set forth in the claims. To particularly point out and distinctly claim the subject matter regarded as invention, the following claims conclude this specification.

## Claims

**1.** A compound of the Formula I

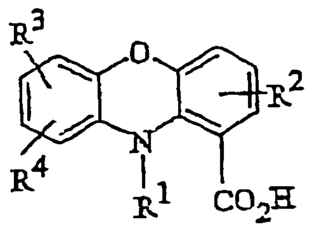

I

and pharmaceutically acceptable salts, esters, wherein the esters are selected from $C_1$-$C_6$ alkyl esters, $C_5$-$C_7$ cycloalkyl esters or arylalkyl esters wherein arylalkyl is defined as below and amides wherein the amides are selected from amides derived from ammonia, primary $C_1$-$C_6$ alkyl amines and secondary $C_1$-$C_6$ dialkyl amines wherein the alkyl groups are straight or branched chain, in the case of secondary amines, the amine may also be in the form of a 5- or 6-membeted heterocycle containing one nitrogen atom,
thereof,
wherein:

$R^1$ is
hydrogen,
$C_1$-$C_6$ alkyl which can be substituted with one or more of the substituents listed below for aryl, or
cycloalkyl, wherein cycloalkyl means a carbocyclic ring having from 3 to 7 carbon atoms which may be substituted with one or more of the substituents listed below for aryl.

$R^2$ is
hydrogen;
$C_1$-$C_6$ Alkyl,
$C_1$-$C_6$ alkoxy,
halogen,
hydroxy,
aryl, wherein aryl means phenyl, biphenyl, 1,2,3,4-tetrahydronaphthyl, naphthyl, anthryl, or phenanthryl, unsubstituted or substituted by 1 to 3 substituents selected from alkyl, O-alkyl and S-alkyl, OH, SH, -CN, halogen, 1,3-dioxolanyl, $CF_3$, $NO_2$, $NH_2$, $NHCH_3$, $N(CH_3)_2$, NHCO-alkyl, $-(CH_2)_mCO_2H$, $-(CH_2)_mCO_2$-alkyl, $-(CH_2)_mSO_3H$, -NH alkyl,-N(alkyl)$_2$, $-(CH_2)_mPO_3H_2$, $-(CH_2)_mPO_3(alkyl)_2$, $-(CH_2)_mSO_2NH_2$, and-$(CH_2)_mSO_2NH$-alkyl wherein alkyl is defined as above and m is 0,1,2, or 3,
heteroaryl, wherein heteroaryl means thienyl, furanyl, thiazolyl, imidazolyl, (is)oxazolyl, pyridyl, pyrimidinyl, (iso)quinolinyl, naphthyridinyl, benzimidazolyl, benzoxazolyl, and heterocycle is unsubstituted or substituted by 1 to 3 substituents selected from alkyl, O-alkyl and S-alkyl, OH, SH, -CN, halogen, 1,3-dioxolanyl, $CF_3$, $NO_2$, $NH_2$, $NHCH_3$, $N(CH_3)_2$, NHCO-alkyl, $-(CH_2)_mCO_2H$, $-(CH_2)_mCO_2$-alkyl,$-(CH_2)_mSO_3H$, -NH alkyl, -N (alkyl)$_2$, $-(CH_2)_mPO_3H_2$,$-(CH_2)_mPO_3(alkyl)_2$, $-(CH_2)_mSO_2NH_2$, and $-(CH_2)_mSO_2NH$-alkyl wherein alkyl is defined as above and m is 0,1, 2, or 3,

arylalkyl, wherein arylalkyl means an alkyl moiety defined as above substituted with an aryl moiety also defined as above,

heteroarylalkyl, wherein heteroaryl and alkyl are defined as above, arylalkoxy, wherein aryl and alkoxy are defined as above, heteroarylalkoxy, wherein heteroaryl and alkoxy are defined as above,

cyano,
carboxy,
alkoxycarbonyl, wherein alkoxy is defined as above,
carbamoyl,
sulfamoyl,
nitro,
trifluoromethyl,
amino, or mono- or dialkylamino wherein alkyl is defined as above;

$R^3$ and $R^4$ independently are

hydrogen,
$C_1$-$C_6$ alkoxy,
aryl defined as above,
heteroaryl defined as above,
halogen,
hydroxy,
cyano,
carboxy,
alkoxycarbonyl defined as above,
carbamoyl,
sulfamoyl,
nitro,
trifluoromethyl,
amino, mono- or dialkylamino defined as above, or

$C_1$-$C_6$ alkyl or alkenyl having up to 6 carbon atoms unsubstituted or substituted with one, two or three groups independently selected from oxo, halogen, hydroxy, carboxy, carbamoyl, amino, mono- or dialkylamino defined as above, or

aryl defined as above or heteroaryl defined as above optionally substituted independently with up to three groups selected from halogen, $C_1$-$C_6$ alkyl, $C_1$-$C_6$ alkoxy, hydroxy, carboxy, alkoxycarbonyl defined as above, cyano, nitro, trifluoromethyl, amino, mono- or dialkylamino defined as above, carbamoyl, carboxyalkyl defined as above, alkoxycarbonylalkyl defined as above, sulfamoyl, or carbonylamino, or

$R^3$ and $R^4$ together form a carbocyclic group containing from five to seven members, up to two of which members are optionally heteroatoms selected from oxygen and nitrogen, where the carbocyclic group is optionally substituted with one or two groups selected from halogen, $C_1$-$C_6$ alkyl,

$C_1$-$C_6$ alkoxy, mono- or dialkylamino defined as above, aryl defined as above, arylalkyl defined as above, or a heterocyclic group defined as above.

**2.** A compound according to claim 1 of the Formula II

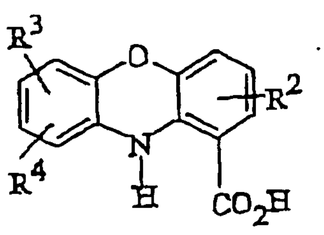

II

and pharmaceutically acceptable salts, esters defined as in claim 1 and amides defined as in claim 1, thereof, wherein:

$R^2$ is hydrogen, $C_1$-$C_6$ alkyl, $C_1$-$C_6$ alkoxy, halogen, hydroxy, aryl defined as in claim 1, heteroaryl defined as in claim 1, arylalkyl defined as in claim 1, heteroarylalkyl defined as in claim 1, arylalkoxy defined as in claim 1, heteroarylalkoxy defined as in claim 1, cyano, carboxy, alkoxycarbonyl defined as in claim 1, carbamoyl,

sulfamoyl, nitro, trifluoromethyl, amino, or mono- or dialkylamino defined as in claim 1;

$R^3$and $R^4$ independently are hydrogen, $C_1$-$C_6$ alkoxy, aryl defined as in claim 1, heteroaryl defined as in claim 1, halogen, hydroxy, cyano, carboxy, alkoxycarbonyl defined as in claim 1, carbamoyl, sulfamoyl, nitro, trifluoromethyl, amino, mono- or dialkylamino defined as in claim 1, or $C_1$-$C_6$ alkyl or alkenyl defined as in claim 1 unsubstituted or substituted with one, two or three groups independently selected from oxo, helogen, hydroxy, carboxy, carbamoyl, amino, mono- or dialkylamino defined as in claim 1, or
aryl defined as in claim 1 or heteroaryl defined as in claim 1 optionally substituted independently with up to three groups selected from halogen, $C_1$-alkyl, $C_1$-$C_6$ alkoxy, hydroxy, carboxy, alkoxycarbonyl defined as in claim 1, cyano, nitro, trifluoromethyl, amino, mono- or dialkylamino defined as in claim 1, carbamoyl, carboxyalkyl defined as in claim 1, alkoxycarbonylalkyl defined as in claim 1, sulfamoyl, or carbonylamino, or

$R^3$ and $R^4$ together form a carbocyclic group containing from five to seven members, up to two of which members are optionally heteroatoms selected from oxygen and nitrogen, where the carbocyclic group is optionally substituted with one or two groups selected from halogen, $C_1$-$C_6$ alkyl, $C_1$-$C_6$ alkoxy, mono- or dialkylamino defined as in claim 1, aryl defined as in claim 1, arylalkyl defined as in claim 1, or a heterocyclic group defined as in claim 1.

**3.** A compound of the Formula III

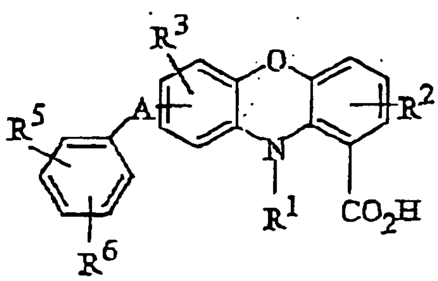

III

and pharmaceutically acceptable salts, esters defined as in claim 1 and amides defined as in claim 1, thereof, wherein:

A is absent, or is
$C_1$-$C_6$ Alkyl or lower alkenyl defined as in claim 1 unsubstituted or substituted with one or two groups independently selected from oxo, halogen, hydroxy, carboxy, carbamoyl, amino, mono- or dialkylamino, defined as in claim 1;

$R^1$ is hydrogen or $C_1$-$C_6$ alkyl;

$R^2$, $R^5$, and $R^6$ are independently hydrogen, $C_1$-$C_6$ alkyl, $C_1$-$C_6$ alkoxy, halogen, hydroxy, aryl defined as in claim 1, heteroaryl defined as in claim 1, arylalkyl defined as in claim 1, heteroarylalkyl defined as in claim 1, arylalkoxy defined as in claim 1, heteroarylalkoxy defined as in claim 1, cyano, carboxy, alkoxycarbonyl defined as in claim 1, carbamoyl, sulfamoyl, nitro, trifluoromethyl, amino, or mono- or dialkylamino defined as in claim 1; and

$R^3$ is hydrogen, $C_1$-$C_6$ alkoxy, aryl defined as in claim 1, heteroaryl defined as in claim 1, halogen, hydroxy, cyano, carboxy, alkoxycarbonyl defined as in claim 1, carbamoyl, sulfamoyl, nitro, trifluoromethyl, amino, mono- or dialkylamino defined as in claim 1, or
$C_1$-$C_6$ alkyl or alkenyl defined as in claim 1 unsubstituted or substituted with one, two or three groups independently selected from oxo, halogen, hydroxy, carboxy, carbamoyl, amino, mono- or dialkylamino defined as in claim 1, or
aryl defined as in claim 1 or heteroaryl defined as in claim 1 optionally substituted independently with up to three groups selected from halogen, $C_1$-$C_6$ alkyl, $C_1$-$C_6$ alkoxy, hydroxy, carboxy, alkoxycarbonyl defined as in claim 1, cyano, nitro, trifluoromethyl, amino, mono- or dialkylamino defined as in claim 1, carbamoyl, carboxyalkyl defined as in claim 1, alkoxycarbonylalkyl defined as in claim 1, sulfamoyl, or carbonylamino.

**4.** A compound of the formula IV

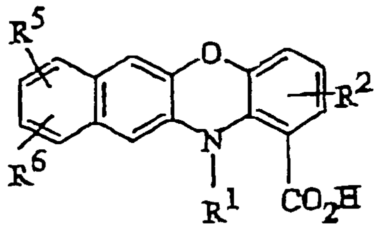

IV

and pharmaceutically acceptable salts, esters defined as in claim 1 and amides defined as in claim 1 thereof, wherein:

$R^1$ is hydrogen or $C_1$-$C_6$ alkyl; and
$R^2$, $R^5$, and $R^6$ are independently hydrogen, $C_1$-$C_6$ alkyl, $C_1$-$C_6$ alkoxy, halogen, hydroxy, aryl defined as in claim 1, heteroaryl defined as in claim 1, arylalkyl defined as in claim 1, heteroarylalkyl defined as in claim 1, arylalkoxy defined as in claim 1, heteroarylalkoxy defined as in claim 1, cyano, carboxy, alkoxycarbonyl defined as in claim 1, carbamoyl, sulfamoyl, nitro, trifluoromethyl, amino, or mono- or dialkylamino defined as in claim 1.

**5.** A compound according to Claim 1, which is selected from:

Phenoxazinecarboxylic acid;
3-Nitrophenoxazinecarboxylic acid;
3-(Phenylmethoxy)phenoxazinecarboxylic acid;
Benzo[b]phenoxazinecarboxylic acid;
8,9-Dimethylbenzo[b]phenoxazinecarboxylic acid;
8,9-Dichlorobenzo[b]phenoxazinecarboxylic acid;
7-Phenylphenoxazinecarboxylic acid;
7-(3,4-Dichlorophenyl)phenoxazinecarboxylic acid;
8-(3,4-Dichlorophenyl)phenoxazinecarboxylic acid;
3-Nitrobenzo[b]phenoxazinecarboxylic acid;
3-Nitro-8-phenylphenoxazinecarboxylic acid;

**6.** Use of a compound of Claim 1 for the manufacturing of pharmaceuticals for the treatment of Alzheimer's disease.

**7.** Use of a compound of Claim 1 for the manufacturing of pharmaceuticals for the inhibition of the aggregation of amyloid proteins to form amyloid deposits.

**8.** Use of a labeled compound of Claim 1 for the manufacturing of diagnostics for the imaging of amyloid deposits.

**9.** Use of a labeled compound of Claim 1 for the manufacturing of diagnostics for the detection of Alzheimer's disease.

**10.** The Use of Claim 8 wherein the labeled compound is a radio labeled compound.

**11.** The use of Claim 8 wherein the labeled compound is to be detected using MRI.

**12.** A compound according to claim 3, which is selected from
7-Benzylphenoxazinecarboxylic acid;
7-[(3,4-Dichlorophenyl)methyl]phenoxazinecarboxylic acid;
7-[2-(3,4-Dichlorophenyl)ethyl]phenoxazinecarboxylic acid;
7-[2-(3,4-Dichlorophenyl)ethyl]-3-nitrophenoxazinecarboxylic acid;
7-[3-(3,4-Dichlorophenyl)-3-oxoprop-1-enyl]-3-nitrophenoxazinecarboxylic acid;
7-[3-(3,4-Dichlorophenyl)propyl]-3-nitrophenoxazine carboxylic acid;
7-[3-(3,4-Dichlorophenyl)-3-hydroxypropyl]-3-nitrophenoxazine carboxylic acid; and
3-Amino-7-[3-(3,4-dichlorophenyl)propyl]phenoxazine carboxylic acid.

**13.** A compound according to claim 4, which is selected from

9-Chloro-8-(trifluoromethyl)benzo[b]phenoxazinecarboxylic acid;
8,9-Dihydroxybenzo[b]phenoxazinecarboxylic acid;

## Patentansprüche

1. Verbindung der Formel I

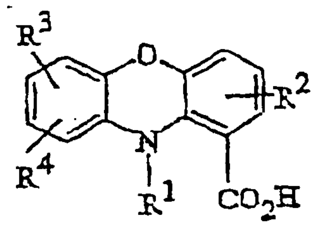

I

und pharmazeutisch akzeptable Salze, Ester, wobei die Ester aus $C_1$-$C_6$-Alkylestern, $C_5$-$C_7$-Cycloalkylestern oder Arylalkylestern, wobei Arylalkyl wie im folgenden definiert ist, ausgewählt sind, und Amide, wobei die Amide aus Amiden, die von Ammoniak, primären $C_1$-$C_6$-Alkylaminen und sekundären $C_1$-$C_6$-Dialkylaminen, wobei die Alkylgruppen gerad- oder verzweigtkettig sind, im Falle sekundärer Amine das Amin auch in der Form eines ein Stickstoffatom enthaltenden 5- oder 6-gliedrigen Heterocyclus vorliegen kann, abgeleitet sind, ausgewählt sind, derselben, worin:

$R^1$ Wasserstoff,
$C_1$-$C_6$-Alkyl, das mit einem oder mehreren der im folgenden für Aryl aufgelisteten Substituenten substituiert sein kann, oder
Cycloalkyl, wobei Cycloalkyl einen carbocyclischen Ring mit 3 bis 7 Kohlenstoffatomen, die mit einem oder mehreren der im folgenden für Aryl aufgelisteten Substituenten substituiert sein können, bedeutet, ist;

$R^2$ Wasserstoff,
$C_1$-$C_6$-Alkyl,
$C_1$-$C_6$-Alkoxy,
Halogen,
Hydroxy,
Aryl, wobei Aryl Phenyl, Diphenyl, 1,2,3,4-Tetrahydronaphthyl, Naphthyl, Anthryl oder Phenanthryl bedeutet, das unsubstituiert oder mit 1 bis 3 Substituenten substituiert ist, die aus Alkyl, O-Alkyl und S-Alkyl, OH, SH, -CN, Halogen, 1,3-Dioxolanyl, $CF_3$, $NO_2$, $NH_2$, $NHCH_3$, $N(CH_3)_2$, NHCO-Alkyl, $-(CH_2)_mCO_2H$, $-(CH_2)_mCO_2$-Alkyl, $-(CH_2)_mSO_3H$, -NH-Alkyl, $-N(Alkyl)_2$, $-(CH_2)_mPO_3H_2$, $-(CH_2)_mPO_3(Alkyl)_2$, $-(CH_2)_mSO_2NH_2$ und $-(CH_2)_mSO_2NH$-Alkyl, wobei Alkyl wie oben definiert ist und m 0, 1, 2 oder 3 ist, ausgewählt sind,
Heteroaryl, wobei Heteroaryl Thienyl, Furanyl, Thiazolyl, Imidazolyl, (Is)oxazolyl, Pyridyl, Pyrimidinyl, (Iso) chinolinyl, Naphthyridinyl, Benzimidazolyl, Benzoxazolyl bedeutet und der Heterocyclus unsubstituiert oder mit 1 bis 3 Substituenten substituiert ist, die aus Alkyl, O-Alkyl und S-Alkyl, OH, SH, -CN, Halogen, 1,3-Dioxolanyl, $CF_3$, $NO_2$, $NH_2$, $NHCH_3$, $N(CH_3)_2$, NHCO-Alkyl, $-(CH_2)_mCO_2H$, $-(CH_2)_mCO_2$-Alkyl, $-(CH_2)_mSO_3H$, -NH-Alkyl, $-N(Alkyl)_2$, $-(CH_2)_mPO_3H_2$, $-(CH_2)_mPO_3(Alkyl)_2$, $-(CH_2)_mSO_2NH_2$ und $-(CH_2)_mSO_2NH$-Alkyl, wobei Alkyl wie oben definiert ist und m 0, 1, 2 oder 3 ist, ausgewählt sind,
Arylalkyl, wobei Arylalkyl eine wie oben definierte Alkyleinheit bedeutet, die mit einer ebenfalls wie oben definierten Aryleinheit substituiert ist, Heteroarylalkyl, wobei Heteroaryl und Alkyl wie oben definiert sind,
Arylalkoxy, wobei Aryl und Alkoxy wie oben definiert sind,
Heteroarylalkoxy, wobei Heteroaryl und Alkoxy wie oben definiert sind,
Cyano,
Carboxy,
Alkoxycarbonyl, wobei Alkoxy wie oben definiert ist, Carbamoyl,
Sulfamoyl,
Nitro,
Trifluormethyl,
Amino oder Mono- oder Dialkylamino, wobei Alkyl wie oben definiert ist, bedeutet;

$R^3$ und $R^4$ unabhängig voneinander
Wasserstoff,
$C_1$-$C_6$-Alkoxy,
Aryl gemäß der obigen Definition,
Heteroaryl gemäß der obigen Definition,
Halogen,
Hydroxy,
Cyano,
Carboxy,
Alkoxycarbonyl gemäß der obigen Definition,
Carbamoyl,
Sulfamoyl,
Nitro,
Trifluormethyl,
Amino, Mono- oder Dialkylamino gemäß der obigen Definition oder
$C_1$-$C_6$-Alkyl oder Alkenyl mit bis zu 6 Kohlenstoffatomen, die unsubstituiert oder mit einer, zwei oder drei Gruppen substituiert sind, die unabhängig voneinander aus Oxo, Halogen, Hydroxy, Carboxy, Carbamoyl, Amino, Mono- oder Dialkylamino gemäß der obigen Definition ausgewählt sind, oder
Aryl gemäß der obigen Definition oder Heteroaryl gemäß der obigen Definition, die optional unabhängig voneinander mit bis zu drei Gruppen substituiert sind, die aus Halogen, $C_1$-$C_6$-Alkyl, $C_1$-$C_6$-Alkoxy, Hydroxy, Carboxy, Alkoxycarbonyl gemäß der obigen Definition, Cyano, Nitro, Trifluormethyl, Amino, Mono- oder Dialkylamino gemäß der obigen Definition, Carbamoyl, Carboxyalkyl gemäß der obigen Definition, Alkoxycarbonylalkyl gemäß der obigen Definition, Sulfamoyl oder Carbonylamino ausgewählt sind, bedeuten, oder

$R^3$ und $R^4$ zusammen eine 5 bis 7 Glieder enthaltende carbocyclische Gruppe bilden, wobei bis zu zwei dieser Glieder optional Heteroatome sind, die aus Sauerstoff und Stickstoff ausgewählt sind, wobei die carbocyclische Gruppe optional mit einer oder zwei Gruppen substituiert ist, die aus Halogen, $C_1$-$C_6$-Alkyl, $C_1$-$C_6$-Alkoxy, Mono- oder Dialkylamino gemäß der obigen Definition, Aryl gemäß der obigen Definition, Arylalkyl gemäß der obigen Definition oder einer heterocyclischen Gruppe gemäß der obigen Definition ausgewählt sind.

**2.** Verbindung gemäß Anspruch 1 der Formel II

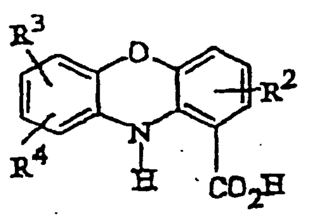

II

und pharmazeutisch akzeptable Salze, Ester gemäß der Definition in Anspruch 1 und Amide gemäß der Definition in Anspruch 1 derselben, worin:

$R^2$ Wasserstoff, $C_1$-$C_6$-Alkyl, $C_1$-$C_6$-Alkoxy, Halogen, Hydroxy, Aryl gemäß der Definition in Anspruch 1, Heteroaryl gemäß der Definition in Anspruch 1, Arylalkyl gemäß der Definition in Anspruch 1, Heteroarylalkyl gemäß der Definition in Anspruch 1, Arylalkoxy gemäß der Definition in Anspruch 1,
Heteroarylalkoxy gemäß der Definition in Anspruch 1, Cyano, Carboxy, Alkoxycarbonyl gemäß der Definition in Anspruch 1, Carbamoyl, Sulfamoyl, Nitro, Trifluormethyl, Amino oder Mono- oder Dialkylamino gemäß der Definition in Anspruch 1 bedeutet;

$R^3$ und $R^4$ unabhängig voneinander
Wasserstoff, $C_1$-$C_6$-Alkoxy, Aryl gemäß der Definition in Anspruch 1, Heteroaryl gemäß der Definition in Anspruch 1, Halogen, Hydroxy, Cyano, Carboxy, Alkoxycarbonyl gemäß der Definition in Anspruch 1, Carbamoyl, Sulfamoyl, Nitro, Trifluormethyl, Amino, Mono- oder Dialkylamino gemäß der Definition in Anspruch 1 oder $C_1$-$C_6$-Alkyl oder Alkenyl gemäß der Definition in Anspruch 1, die unsubstituiert oder mit einer, zwei oder drei Gruppen substituiert sind, die unabhängig voneinander aus Oxo, Halogen, Hydroxy, Carboxy, Carbamoyl, Amino, Mono- oder Dialkylamino gemäß der Definition in Anspruch 1 ausgewählt sind, oder

Aryl gemäß der Definition in Anspruch 1 oder Heteroaryl gemäß der Definition in Anspruch 1, die optional unabhängig voneinander mit bis zu drei Gruppen substituiert sind, die aus Halogen, $C_1$-$C_6$-Alkyl, $C_1$-$C_6$-Alkoxy, Hydroxy, Carboxy, Alkoxycarbonyl gemäß der Definition in Anspruch 1, Cyano, Nitro, Trifluormethyl, Amino, Mono- oder Dialkylamino gemäß der Definition in Anspruch 1, Carbamoyl, Carboxyalkyl gemäß der Definition in Anspruch 1, Alkoxycarbonylalkyl gemäß der Definition in Anspruch 1, Sulfamoyl oder Carbonylamino ausgewählt sind, bedeuten, oder

$R^3$ und $R^4$ zusammengenommen eine 5 bis 7 Glieder enthaltende carbocyclische Gruppe bilden, wobei bis zu zwei dieser Glieder optional Heteroatome sind, die aus Sauerstoff und Stickstoff ausgewählt sind, wobei die carbocyclische Gruppe optional mit einer oder zwei Gruppen substituiert ist, die aus Halogen, $C_1$-$C_6$-Alkyl, $C_1$-$C_6$-Alkoxy, Mono- oder Dialkylamino gemäß der Definition in Anspruch 1, Aryl gemäß der Definition in Anspruch 1, Aralkyl gemäß der Definition in Anspruch 1 oder einer heterocyclischen Gruppe gemäß der Definition in Anspruch 1 ausgewählt sind.

**3.** Verbindung der Formel III

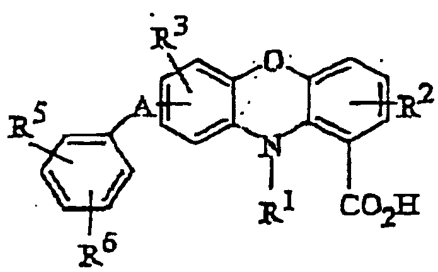

III

und pharmazeutisch akzeptable Salze, Ester gemäß der Definition in Anspruch 1 und Amide gemäß der Definition in Anspruch 1 derselben, worin:

A nicht vorhanden ist oder
$C_1$-$C_6$-Alkyl oder Niederalkenyl gemäß der Definition in Anspruch 1, die unsubstituiert oder mit einer oder zwei Gruppen substituiert sind, die unabhängig voneinander aus Oxo, Halogen, Hydroxy, Carboxy, Carbamoyl, Amino, Mono- oder Dialkylamino gemäß der Definition in Anspruch 1 ausgewählt sind, bedeutet;

$R^1$ Wasserstoff oder $C_1$-$C_6$-Alkyl bedeutet;

$R^2$, $R^5$ und $R^6$ unabhängig voneinander Wasserstoff, $C_1$-$C_6$-Alkyl, $C_1$-$C_6$-Alkoxy, Halogen, Hydroxy, Aryl gemäß der Definition in Anspruch 1, Heteroaryl gemäß der Definition in Anspruch 1, Arylalkyl gemäß der Definition in Anspruch 1, Heteroarylalkyl gemäß der Definition in Anspruch 1, Arylalkoxy gemäß der Definition in Anspruch 1, Heteroarylalkoxy gemäß der Definition in Anspruch 1, Cyano, Carboxy, Alkoxycarbonyl gemäß der Definition in Anspruch 1, Carbamoyl, Sulfamoyl, Nitro, Trifluormethyl, Amino oder Mono- oder Dialkylamino gemäß der Definition in Anspruch 1 bedeuten; und

$R^3$ Wasserstoff, $C_1$-$C_6$-Alkoxy, Aryl gemäß der Definition in Anspruch 1, Heteroaryl gemäß der Definition in Anspruch 1, Halogen, Hydroxy, Cyano, Carboxy, Alkoxycarbonyl gemäß der Definition in Anspruch 1, Carbamoyl, Sulfamoyl, Nitro, Trifluormethyl, Amino, Mono- oder Dialkylamino gemäß der Definition in Anspruch 1 oder
$C_1$-$C_6$-Alkyl oder Alkenyl gemäß der Definition in Anspruch 1, die unsubstituiert oder mit einer, zwei oder drei Gruppen substituiert sind, die unabhängig voneinander aus Oxo, Halogen, Hydroxy, Carboxy, Carbamoyl, Amino, Mono- oder Dialkylamino gemäß der Definition in Anspruch 1 ausgewählt sind, oder
Aryl gemäß der Definition in Anspruch 1 oder Heteroaryl gemäß der Definition in Anspruch 1, die optional unabhängig voneinander mit bis zu drei Gruppen substituiert sind, die aus Halogen, $C_1$-$C_6$-Alkyl, $C_1$-$C_6$-Alkoxy, Hydroxy, Carboxy, Alkoxycarbonyl gemäß der Definition in Anspruch 1, Cyano, Nitro, Trifluormethyl, Amino, Mono- oder Dialkylamino gemäß der Definition in Anspruch 1, Carbamoyl, Carboxyalkyl gemäß der Definition in Anspruch 1, Alkoxycarbonylalkyl gemäß der Definition in Anspruch 1, Sulfamoyl oder Carbonylamino ausgewählt sind, bedeutet.

4. Verbindung der Formel IV

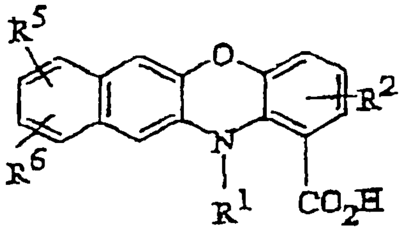

IV

und pharmazeutisch akzeptable Salze, Ester gemäß der Definition in Anspruch 1 und Amide gemäß der Definition in Anspruch 1 derselben, worin:

$R^1$ Wasserstoff oder $C_1$-$C_6$-Alkyl bedeutet; und

$R^2$, $R^5$ und $R^6$ unabhängig voneinander Wasserstoff, $C_1$-$C_6$-Alkyl, $C_1$-$C_6$-Alkoxy, Halogen, Hydroxy, Aryl gemäß der Definition in Anspruch 1, Heteroaryl gemäß der Definition in Anspruch 1, Arylalkyl gemäß der Definition in Anspruch 1, Heteroarylalkyl gemäß der Definition in Anspruch 1, Arylalkoxy gemäß der Definition in Anspruch 1, Heteroarylalkoxy gemäß der Definition in Anspruch 1, Cyano, Carboxy, Alkoxycarbonyl gemäß der Definition in Anspruch 1, Carbamoyl, Sulfamoyl, Nitro, Trifluormethyl, Amino oder Mono- oder Dialkylamino gemäß der Definition in Anspruch 1 bedeuten.

5. Verbindung nach Anspruch 1, die aus
Phenoxazincarbonsäure,
3-Nitrophenoxazincarbonsäure,
3-(Phenylmethoxy)phenoxazincarbonsäure,
Benzo[b]phenoxazincarbonsäure,
8,9-Dimethylbenzo[b]phenoxazincarbonsäure,
8,9-Dichlorbenzo[b]phenoxazincarbonsäure,
7-Phenylphenoxazincarbonsäure,
7-(3,4-Dichlorphenyl)phenoxazincarbonsäure,
8-(3,4-Dichlorphenyl)phenoxazincarbonsäure,
3-Nitrobenzo[b]phenoxazincarbonsäure,
3-Nitro-8-phenylphenoxazincarbonsäure ausgewählt ist.

6. Verwendung einer Verbindung nach Anspruch 1 zur Herstellung von Pharmazeutika zur Behandlung von Alzheimer-Krankheit.

7. Verwendung einer Verbindung nach Anspruch 1 zur Herstellung von Pharmazeutika zur Hemmung der Aggregation von Amyloidproteinen unter Bildung von Amyloidablagerungen.

8. Verwendung einer markierten Verbindung nach Anspruch 1 zur Herstellung von Diagnostika zur Bildgebung von Amyloidablagerungen.

9. Verwendung einer markierten Verbindung nach Anspruch 1 zur Herstellung von Diagnostika zur Detektion von Alzheimer-Krankheit.

10. Verwendung nach Anspruch 8, wobei die markierte Verbindung eine radioaktiv markierte Verbindung ist.

11. Verwendung nach Anspruch 8, wobei die markierte Verbindung unter Verwendung von MRI zu detektieren ist.

12. Verbindung nach Anspruch 3, die aus
7-Benzylphenoxazincarbonsäure,
7-[(3,4-Dichlorphenyl)methyl]phenoxazincarbonsäure,
7-[2-(3,4-Dichlorphenyl)ethyl]phenoxazincarbonsäure,
7-[2-(3,4-Dichlorphenyl)ethyl]-3-nitrophenoxazincarbonsäure,

7-[3-(3,4-Dichlorphenyl)-3-oxoprop-1-enyl]-3-nitrophenoxazincarbonsäure,
7-[3-(3,4-Dichlorphenyl)propyl]-3-nitrophenoxazincarbonsäure,
7-[3-(3,4-Dichlorphenyl)-3-hydroxypropyl]-3-nitrophenoxazincarbonsäure und
3-Amino-7-[3-(3,4-dichlorphenyl)propyl]phenoxazincarbonsäure ausgewählt ist.

**13.** Verbindung nach Anspruch 4, die aus
9-Chlor-8-(trifluormethyl)benzo[b]phenoxazincarbonsäure,
8,9-Dihydroxybenzo[b]phenoxazincarbonsäure ausgewählt ist.

## Revendications

**1.** Composé répondant à la formule I

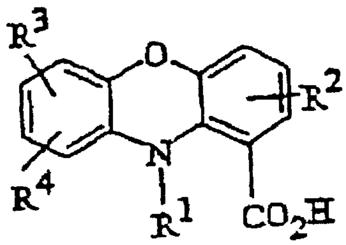

I

et sels pharmaceutiquement acceptables, esters, dans lesquels les esters sont choisis parmi les esters alkyliques en $C_1$-$C_6$, les esters cycloalkyliques en $C_5$-$C_7$ ou les esters arylalkyliques dans lesquels le groupe arylalkyle est défini comme ci-dessous, et amides dans lesquels les amides sont choisis parmi les amides dérivés de l'ammoniac, de (alkyle en $C_1$-$C_6$)amines primaires et de di(alkyle en $C_1$-$C_6$)amines secondaires dans lesquelles les groupes alkyles sont des chaînes linéaires ou ramifiées, dans le cas des amines secondaires, l'amine peut également être sous la forme d'un hétérocyclique de 5 ou 6 éléments contenant un atome d'hydrogène,
de celui-ci,
dans lequel
$R^1$ représente
un atome d'hydrogène,
un groupe alkyle en $C_1$-$C_6$ qui peut être substitué par un ou plusieurs des substituants énumérés ci-dessous pour le groupe aryle, ou
un groupe cycloalkyle, dans lequel le terme cycloalkyle signifie un cycle carbocyclique ayant de 3 à 7 atomes de carbone qui peut être substitué par un ou plusieurs des substituants énumérés ci-dessous pour le groupe aryle ;
$R^2$ représente
un atome d'hydrogène,
un groupe alkyle en $C_1$-$C_6$,
un groupe alcoxy en $C_1$-$C_6$,
un atome d'halogène,
un groupe hydroxy,
un groupe aryle, dans lequel le terme aryle signifie un groupe phényle, un groupe biphényle, un groupe 1,2,3,4-tétrahydronaphtyle, un groupe naphtyle, un groupe anthryle, ou un groupe phénanthryle, non substitué ou substitué par 1 à 3 substituants choisis parmi un groupe alkyle, un groupe O-alkyle et un groupe S-alkyle, OH, SH, -CN, un atome d'halogène, un groupe 1,3-dioxolanyle, $CF_3$, $NO_2$, $NH_2$, $NHCH_3$, $N(CH_3)_2$, NHCO-alkyle, $-(CH_2)_mCO_2H$, $-(CH_2)_mCO_2$-alkyle, $-(CH_2)_mSO_3H$, -NH-alkyle, -N(alkyle)$_2$, $-(CH_2)_mPO_3H_2$, $-(CH_2)_mPO_3$(alkyle)$_2$, $-(CH_2)_mSO_2NH_2$, et $-(CH_2)_mSO_2$NH-alkyle, dans lesquels le groupe alkyle est défini comme ci-dessus et m vaut 0, 1, 2, ou 3,
un groupe hétéroaryle, dans lequel le terme hétéroaryle signifie un groupe thiényle, un groupe furanyle, un groupe thiazolyle, un groupe imidazolyle, un groupe (is)oxazolyle, un groupe pyridyle, un groupe pyrimidinyle, un groupe (iso)quinoléinyle, un groupe naphtyridinyle, un groupe benzimidazolyle, un groupe benzoxazolyle, et l'hétérocycle est non substitué ou substitué par 1 à 3 substituants choisis parmi un groupe alkyle, un groupe O-alkyle et un groupe S-alkyle, OH, SH, -CN, un atome d'halogène, un groupe 1,3-dioxolanyle, $CF_3$, $NO_2$, $NH_2$, $NHCH_3$, $N(CH_3)_2$, NHCO-alkyle, $-(CH_2)_mCO_2H$, $-(CH_2)_mCO_2$-alkyle, $-(CH_2)_mSO_3H$, -NH-alkyle, -N(alkyle)$_2$, $-(CH_2)_mPO_3H_2$, $-(CH_2)_mPO_3$(alkyle)$_2$, $-(CH_2)_mSO_2NH_2$, et $-(CH_2)_mSO_2$NH-alkyle, dans lesquels le groupe alkyle est défini comme

ci-dessus et m vaut 0, 1, 2, ou 3,

un groupe arylalkyle, dans lequel le terme arylalkyle signifie un fragment alkyle défini comme ci-dessus substitué par un fragment aryle également défini comme ci-dessus,

un groupe hétéroarylalkyle, dans lequel hétéroaryle et alkyle sont définis comme ci-dessus,

un groupe arylalcoxy, dans lequel aryle et alcoxy sont définis comme ci-dessus,

un groupe hétéroarylalcoxy, dans lequel hétéroaryle et alcoxy sont définis comme ci-dessus,

un groupe cyano,

un groupe carboxy,

un groupe alcoxycarbonyle, dans lequel alcoxy est défini comme ci-dessus,

un groupe carbamoyle,

un groupe sulfamoyle,

un groupe nitro,

un groupe trifluorométhyle,

un groupe amino, ou un groupe mono- ou dialkylamino, dans lequel alkyle est défini comme ci-dessus ;

$R^3$ et $R^4$ représentent indépendamment

un atome d'hydrogène,

un groupe alcoxy en $C_1$-$C_6$,

un groupe aryle défini comme ci-dessus,

un groupe hétéroaryle défini comme ci-dessus,

un atome d'halogène,

un groupe hydroxy,

un groupe cyano,

un groupe carboxy,

un groupe alcoxycarbonyle défini comme ci-dessus,

un groupe carbamoyle,

un groupe sulfamoyle,

un groupe nitro,

un groupe trifluorométhyle,

un groupe amino, ou un groupe mono- ou dialkylamino défini comme ci-dessus, ou

un groupe alkyle ou alcényle en $C_1$-$C_6$ ayant jusqu'à 6 atomes de carbone non substitué ou substitué par un, deux ou trois groupes indépendamment choisis parmi un groupe oxo, un atome d'halogène, un groupe hydroxy, un groupe carboxy, un groupe carbamoyle, un groupe amino, un groupe mono- ou dialkylamino défini comme ci-dessus, ou

un groupe aryle défini comme ci-dessus ou un groupe hétéroaryle défini comme ci-dessus éventuellement substitué indépendamment par jusqu'à trois groupes choisis parmi un atome d'halogène, un groupe alkyle en $C_1$-$C_6$, un groupe alcoxy en $C_1$-$C_6$, un groupe hydroxy, un groupe carboxy, un groupe alcoxycarbonyle défini comme ci-dessus, un groupe cyano, un groupe nitro, un groupe trifluorométhyle, un groupe amino, un groupe mono- ou dialkylamino défini comme ci-dessus, un groupe carbamoyle, un groupe carboxyalkyle défini comme ci-dessus, un groupe alcoxycarbonylalkyle défini comme ci-dessus, un groupe sulfamoyle, ou un groupe carbonylamino, ou

$R^3$ et $R^4$ ensemble forment un groupe carbocyclique contenant de 5 à 7 éléments, dont deux éléments au plus sont éventuellement des hétéroatomes choisis parmi

un atome d'oxygène et un atome d'azote, où le groupe carbocyclique est éventuellement substitué par un ou deux groupes choisis parmi un atome d'halogène, un groupe alkyle en $C_1$-$C_6$,

un groupe alcoxy en $C_1$-$C_6$, un groupe mono- ou dialkylamino défini comme ci-dessus, un groupe aryle défini comme ci-dessus, un groupe arylalkyle défini comme ci-dessus, ou un groupe hétérocyclique défini comme ci-dessus.

**2.** Composé selon la revendication 1 répondant à la formule II

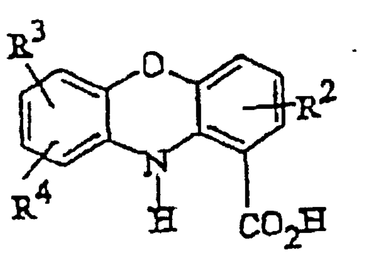

II

et sels pharmaceutiquement acceptables, esters définis comme dans la revendication 1 et amides définis comme dans la revendications 1, de celui-ci,
dans lequel
$R^2$ représente un atome d'hydrogène, un groupe alkyle en $C_1$-$C_6$, un groupe alcoxy en $C_1$-$C_6$, un atome d'halogène, un groupe hydroxy, un groupe aryle défini comme dans la revendication 1, un groupe hétéroaryle défini comme dans la revendication 1, un groupe arylalkyle défini comme dans la revendication 1, un groupe hétéroarylalkyle défini comme dans la revendication 1, un groupe arylalcoxy défini comme dans la revendication 1, un groupe hétéroarylalcoxy défini comme dans la revendication 1, un groupe cyano, un groupe carboxy, un groupe alcoxycarbonyle défini comme dans la revendication 1, un groupe carbamoyle, un groupe sulfamoyle, un groupe nitro, un groupe trifluorométhyle, un groupe amino, ou un groupe mono- ou dialkylamino défini comme dans la revendication 1 ;
$R^3$ et $R^4$ représentent indépendamment un atome d'hydrogène, un groupe alcoxy en $C_1$-$C_6$, un groupe aryle défini comme dans la revendication 1, un groupe hétéroaryle défini comme dans la revendication 1, un atome d'halogène, un groupe hydroxy, un groupe cyano, un groupe carboxy, un groupe alcoxycarbonyle défini comme dans la revendication 1, un groupe carbamoyle, un groupe sulfamoyle, un groupe nitro, un groupe trifluorométhyle, un groupe amino, un groupe mono- ou dialkylamino défini comme dans la revendication 1, ou un groupe alkyle ou alcényle en $C_1$-$C_6$ défini comme dans la revendication 1 non substitué ou substitué par un, deux ou trois groupes indépendamment choisis parmi un groupe oxo, un atome d'halogène, un groupe hydroxy, un groupe carboxy, un groupe carbamoyle, un groupe amino, un groupe mono- ou dialkylamino défini comme dans la revendication 1, ou
un groupe aryle défini comme dans la revendication 1 ou un groupe hétéroaryle défini comme dans la revendication 1 éventuellement substitué indépendamment par trois groupes au plus choisis parmi un atome d'halogène, un groupe alkyle en $C_1$-$C_6$, un groupe alcoxy en $C_1$-$C_6$, un groupe hydroxy, un groupe carboxy, un groupe alcoxycarbonyle défini comme dans la revendication 1, un groupe cyano, un groupe nitro, un groupe trifluorométhyle, un groupe amino, un groupe mono- ou dialkylamino défini comme dans la revendication 1, un groupe carbamoyle, un groupe carboxyalkyle défini comme dans la revendication 1, un groupe alcoxycarbonylalkyle défini comme dans la revendication 1, un groupe sulfamoyle, ou un groupe carbonylamino, ou
$R^3$ et $R^4$ ensemble forment un groupe carbocyclique contenant de 5 à 7 éléments, dont deux éléments au plus sont éventuellement des hétéroatomes choisis parmi un atome d'oxygène et un atome d'azote, où le groupe carbocyclique est éventuellement substitué par un ou deux groupes choisis parmi un atome d'halogène, un groupe alkyle en $C_1$-$C_6$, un groupe alcoxy en $C_1$-$C_6$, un groupe mono- ou dialkylamino défini comme dans la revendication 1, un groupe aryle défini comme dans la revendication 1, un groupe arylalkyle défini comme dans la revendication 1, ou un groupe hétérocyclique défini comme dans la revendication 1.

3. Composé répondant à la formule III

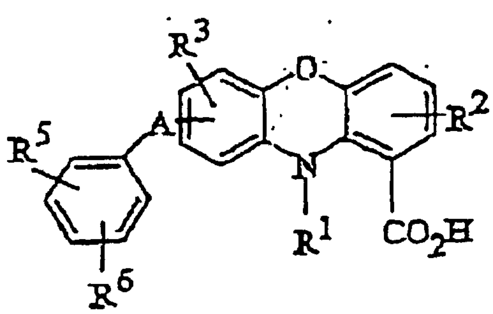

III

et sels pharmaceutiquement acceptables, esters définis comme dans la revendication 1 et amides définis comme dans la revendication 1, de celui-ci,
dans lequel
A est absent ou représente
un groupe alkyle en $C_1$-$C_6$ ou un groupe alcényle inférieur défini comme dans la revendication 1 non substitué ou substitué par un ou deux groupes indépendamment choisis parmi un groupe oxo, un atome d'halogène, un groupe hydroxy, un groupe carboxy, un groupe carbamoyle, un groupe amino, un groupe mono- ou dialkylamino défini comme dans la revendication 1 ;
$R^1$ représente un atome d'hydrogène ou un groupe alkyle en $C_1$-$C_6$ ;
$R^2$, $R^5$, et $R^6$ représentent indépendamment un atome d'hydrogène, un groupe alkyle en $C_1$-$C_6$, un groupe alcoxy en $C_1$-$C_6$, un atome d'halogène, un groupe hydroxy, un groupe aryle défini comme dans la revendication 1, un

groupe hétéroaryle défini comme dans la revendication 1, un groupe arylalkyle défini comme dans la revendication 1, un groupe hétéroarylalkyle défini comme dans la revendication 1, un groupe arylalcoxy défini comme dans la revendication 1, un groupe hétéroarylalcoxy défini comme dans la revendication 1, un groupe cyano, un groupe carboxy, un groupe alcoxycarbonyle défini comme dans la revendication 1, un groupe carbamoyle, un groupe sulfamoyle, un groupe nitro, un groupe trifluorométhyle, un groupe amino, ou un groupe mono- ou dialkylamino défini comme dans la revendication 1 ; et

$R^3$ représente un atome d'hydrogène, un groupe alcoxy en $C_1$-$C_6$, un groupe aryle défini comme dans la revendication 1, un groupe hétérparyle défini comme dans la revendication 1, un atome d'halogène, un groupe hydroxy, un groupe cyano, un groupe carboxy, un groupe alcoxycarbonyle défini comme dans la revendication 1, un groupe carbamoyle, un groupe sulfamoyle, un groupe nitro, un groupe trifluorométhyle, un groupe amino, un groupe mono- ou dialkylamino défini comme dans la revendication 1, ou

un groupe alkyle ou alcényle en $C_1$-$C_6$ défini comme dans la revendication 1 non substitué ou substitué par un, deux ou trois groupes indépendamment choisis parmi un groupe oxo, un atome d'halogène, un groupe hydroxy, un groupe carboxy, un groupe carbamoyle, un groupe amino, un groupe mono- ou dialkylamino défini comme dans la revendication 1, ou

un groupe aryle défini comme dans la revendication 1 ou un groupe hétéroaryle défini comme dans la revendication 1 éventuellement substitué indépendamment par trois groupes au plus choisis parmi un atome d'halogène, un groupe alkyle en $C_1$-$C_6$, un groupe alcoxy en $C_1$-$C_6$, un groupe hydroxy, un groupe carboxy, un groupe alcoxycarbonyle défini comme dans la revendication 1, un groupe cyano, un groupe nitro, un groupe trifluorométhyle, un groupe amino, un groupe mono- ou dialkylamino défini comme dans la revendication 1, un groupe carbamoyle, un groupe carboxyalkyle défini comme dans la revendication 1, un groupe alcoxycarbonylalkyle défini comme dans la revendication 1, un groupe sulfamoyle, ou un groupe carbonylamino.

**4.** Composé répondant à la formule IV

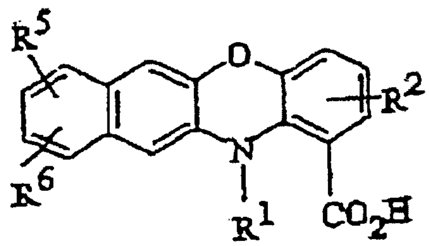

IV

et sels pharmaceutiquement acceptables, esters définis comme dans la revendication 1 et amides définis comme dans la revendication 1, de celui-ci,

dans lequel

$R^1$ représente un atome d'hydrogène ou un groupe alkyle en $C_1$-$C_6$ ; et

$R^2$, $R^5$, et $R^6$ représentent indépendamment un atome d'hydrogène, un groupe alkyle en $C_1$-$C_6$, un groupe alcoxy en $C_1$-$C_6$, un atome d'halogène, un groupe hydroxy, un groupe aryle défini comme dans la revendication 1, un groupe hétéroaryle défini comme dans la revendication 1, un groupe arylalkyle défini comme dans la revendication 1, un groupe hétéroarylalkyle défini comme dans la revendication 1, un groupe arylalcoxy défini comme dans la revendication 1, un groupe hétéroarylalcoxy défini comme dans la revendication 1, un groupe cyano, un groupe carboxy, un groupe alcoxycarbonyle défini comme dans la revendication 1, un groupe carbamoyle, un groupe sulfamoyle, un groupe nitro, un groupe trifluorométhyle, un groupe amino, ou un groupe mono- ou dialkylamino défini comme dans la revendication 1.

**5.** Composé selon la revendication 1, qui est choisi parmi
l'acide phénoxazinecarboxylique ;
l'acide 3-nitrophénoxazinecarboxylique ;
l'acide 3-(phénylméthoxy)phénoxazinecarboxylique ;
l'acide benzo[b]phénoxazinecarboxylique ;
l'acide 8,9-diméthylbenzo[b]phénoxazinecarboxylique ;
l'acide 8,9-dichlorobenzo[b]phénoxazinecarboxylique ;
l'acide 7-phénylphénoxazinecarboxylique ;
l'acide 7-(-3,4-dichlorophényl)phénoxazinecarboxylique ;
l'acide 8-(-3,4-dichlorophényl)phénoxazinecarboxylique ;

l'acide 3-nitrobenzo[b]phénoxazinecarboxylique ;
l'acide 3-nitro-8-phénylphénoxazinecarboxylique.

6. Utilisation d'un composé selon la revendication 1 pour la préparation de produits pharmaceutiques destinés au traitement de la maladie d'Alzheimer.

7. Utilisation d'un composé selon la revendication 1 pour la préparation de produits pharmaceutiques destinés à inhiber l'agrégation de protéines amyloïdes pour former des dépôts d'amyloïdes.

8. Utilisation d'un composé marqué selon la revendication 1 pour la fabrication d'outils de diagnostic pour la présentation en image de dépôts d'amyloïde.

9. Utilisation d'un composé marqué selon la revendication 1 pour la fabrication d'outils de diagnostic pour la détection de la maladie d'Alzheimer.

10. Utilisation selon la revendication 8, dans laquelle le composé marqué est un composé radiomarqué.

11. Utilisation selon la revendication 8, dans laquelle le composé marqué est détecté en utilisant une IRM.

12. Composé selon la revendication 3, qui est choisi parmi
l'acide 7-benzylphénoxazinecarboxylique ;
l'acide 7-[(3,4-dichlorophényl)méthyl]phénoxazinecarboxylique ;
l'acide 7-[2-(3,4-dichlorophényl)éthyl]phénoxazinecarboxylique ;
l'acide 7-[2-(3,4-dichlorophényl)éthyl]-3-nitrophénoxazinecarboxylique ;
l'acide 7-[3-(3,4-dichlorophényl)-3-oxoprop-1-ényl]-3-nitrophénoxazine-carboxylique ;
l'acide 7-[3-(3,4-dichlorophényl)propyl]-3-nitrophénoxazinecarboxylique ;
l'acide 7-[3-(3,4-dichlorophényl)-3-hydroxypropyl]-3-nitrophénoxazinecarboxylique ; et
l'acide 3-amino-7-[3-(3,4-dichlorophényl)propyl]phénoxazinecarboxylique.

13. Composé selon la revendication 4, qui est choisi parmi
l'acide 9-chloro-8-(-trifluorométhyl)benzo[b]phénoxazinecarboxylique ;
l'acide 8,9-dihydroxybenzo[b]phénoxazinecarboxylique.